# EUROPÄISCHE PATENTSCHRIFT

(11) **EP 3 470 001 B1**
(45) Veröffentlichungstag und Bekanntmachung des Hinweises auf die Patenterteilung: **21.04.2021**
(21) Anmeldenummer: 17195784.8
(22) Anmeldetag: 10.10.2017
(51) Int. Cl.: A61B 17/72, A61B 17/04

(54) **IMPLANTAT ZUM ZUGFESTEN VERBINDEN ZUMINDEST ZWEIER TEILE EINES GEBROCHENEN RÖHRENKNOCHENS**
IMPLANT FOR TENSION-RESISTANT CONNECTION OF AT LEAST TWO PARTS OF A BROKEN TUBULAR BONE
IMPLANT DE RACCORDEMENT RÉSISTANT À LA TRACTION D'AU MOINS DEUX PARTIES D'UN OS LONG CASSÉ

(43) Veröffentlichungstag der Anmeldung: 17.04.2019
(73) Patentinhaber: AIT Austrian Institute of Technology GmbH, 1220 Wien (AT); Pieske, Oliver, 26121 Oldenburg (DE)
(72) Erfinder: ETTEL, Walter, 2521 Trumau (AT); GFATTER, Rosemarie, 2700 Wiener Neustadt (AT); HORKY, Jelena, 1070 Wien (AT); PIESKE, Oliver, 26121 Oldenburg (DE)
(74) Vertreter: Wirnsberger & Lerchbaum Patentanwälte OG

(56) Entgegenhaltungen:
- WO-A1-2007/134345
- DE-A1-102009 051 367
- DE-A1-102015 107 056
- US-A- 5 417 692
- US-A1- 2010 036 439
- US-A1- 2014 214 080
- US-A1- 2014 343 616
- US-A1- 2016 038 186
- US-A1- 2017 065 424
- US-B1- 6 458 134

## Beschreibung

Die Erfindung betrifft ein Implantat zum zugfesten Verbinden zweier Teile eines gebrochenen Röhrenknochens, umfassend ein Verbindungselement, zumindest einen Faden und zumindest ein Ankerelement, wobei das zumindest eine Ankerelement mit dem zumindest einen Faden an einem Knochenteil befestigbar ist, wobei das Verbindungselement derart ausgeführt ist, dass dieses in Längsrichtung des gebrochenen Röhrenknochens in einen Markraum des gebrochenen Röhrenknochens einführbar ist, wobei das Verbindungselement zumindest eine Durchgangsöffnung aufweist, durch welche der zumindest eine Faden zum zugfesten Spannen der Röhrenknochenteile durchführbar ist.

Des Weiteren betrifft die Erfindung ein Verfahren zur Herstellung eines derartigen Implantates.

Zur Behandlung von gebrochenen Röhrenknochen sind aus dem Bereich der Osteosynthese verschiedene Implantate bekannt, welche nach einer Reposition der Knochenteile eine Fixierung und Stabilisierung der Knochenteile, als Retention bezeichnet, gewährleisten. Eine minimal-invasive Retention wird insbesondere durch eine intramedulläre Stabilisierung von Röhrenknochenteilen durch das Einbringen einer Schienung in einen Markraum des gebrochenen Röhrenknochens erreicht.

Die DE 38 40 798 A1 beschreibt einen Marknagel, der mit Verriegelungsschrauben fixierbar ist, zur operativen Behandlung von Frakturen langer Röhrenknochen, wobei der Bereich der Nagelspitze aus einem resorbierbaren Material bestehen kann. Eine Fixierung erfolgt durch das Einsetzen von Verriegelungsschrauben in vorgesehene Löcher im Marknagel.

Das Einsetzen eines Marknagels ermöglicht zwar die Schonung des den Bruchbereich umgebenden Gewebes, jedoch weist dieser eine verhältnismäßig geringe Stabilität auf, kann auftretende Dislokationskräfte nur begrenzt kompensieren und birgt die Gefahr einer auftretenden Pseudoarthrose. Bekannt ist weiter, dass durch eine Kompression der Frakturteile aneinander die Stabilität einer Knochenverbindung weiter erhöht werden kann und eine Kompression der gebrochenen Knochenteile außerdem die Frakturheilung stimuliert. Eine solche Kompression wird durch einen Marknagel nicht bewirkt. Um neben einer Stabilisierung außerdem ein Aneinanderpressen der Frakturteile an der Bruchstelle zu erreichen, wurde ein Implantat bestehend aus Formstück und Seil entwickelt.

Die DE 10 2005 023 069 B4 offenbart ein Implantat zum zugfesten Verbinden zweier Teile eines im Wesentlichen glatt gebrochenen Röhrenknochens, wobei das Implantat durch ein Formstück und ein Seil gebildet ist, welches in Längsrichtung eines gebrochenen Röhrenknochens in einen Markraum des gebrochenen Röhrenknochens eingeführt werden kann. Das Formstück weist eine Durchgangsöffnung auf, welche sich durch das gesamte Formstück erstreckt und eine Durchführung des Seiles erlaubt, um die beiden Knochenteile zugfest aneinander zu spannen, indem das Seil mit Ankerstücken an den Knochenteilen fixiert wird. Das Formstück ist dabei aus einem resorbierbaren Material gebildet, um zu verhindern, dass das Formstück wieder aus dem Markraum entfernt werden muss.

Die Eignung eines solchen Implantats mit einem Verbindungselement, welches zwei Teile eines gebrochenen Röhrenknochens intramedullär mechanisch stabilisieren und durch ein Seil zugfest aneinanderpressen soll, ist allerdings dadurch eingeschränkt, dass das Verbindungselement durch einen Frakturspalt hindurch in einen Markraum eines Knochenteiles eingebracht werden muss. Ein solches Einbringen kann sich abhängig von der Größe des Frakturspalts und der Größe bzw. Ausdehnung des einzubringenden Verbindungselementes, vor allem bei eingeschränkter Zugänglichkeit, als schwierig erweisen. Weiter kann es notwendig sein, dass eine Markhöhle für eine Implantation eines Verbindungselementes vorab präpariert werden muss, beispielsweise durch eine Aufweitung und/oder ein Ausbohren der Markhöhle entsprechend der Größe des Verbindungselementes. Im Fall von erschwerter Zugänglichkeit und im Speziellen bei einer länglich ausgedehnten Form des Verbindungselementes kann deshalb ein Einbringen des Verbindungselementes in dem Markraum eines Knochenteils mit einer zusätzlichen Traumatisierung des umliegenden Gewebes verbunden sein.

Weitere Implantate zum zugfesten Verbinden zweier Teile eines gebrochenen Röhrenknochens werden in folgenden Dokumenten offenbart: US 2014/0214080 A1; US 2016/0038186 A1; DE 10 2009 051 367 A1; US 2010/0036439 A1; US 6,458,134 B1; US 5,417,692 A; US 2017/0065424 A1; US 2014/0343616 A1.

Hier setzt die Erfindung an. Aufgabe der Erfindung ist es, ein Implantat der eingangs genannten Art anzugeben, bei welchem die Einsatztauglichkeit erhöht ist.

Weiter ist es ein Ziel, ein Verfahren zur Herstellung eines solchen Implantates anzugeben.

Die Aufgabe wird erfindungsgemäß gelöst durch Implantate gemäß den unabhängigen Ansprüchen 1, 13 und 14 und durch Verfahren zur Herstellung solcher Implantate gemäß den Ansprüchen 15 und 16.

Es kann vorgesehen sein, dass das Verbindungselement zweiteilig ausgebildet ist.

Ein Vorteil ist darin zu sehen, dass durch die zweiteilige Ausbildung des Verbindungselementes ein Einbringen der Verbindungselementteile durch einen Frakturspalt in die beiden Röhrenknochenteile einfacher bewerkstelligt werden kann und eine etwaige hierzu erforderliche zusätzliche Aufweitung des Frakturspaltes nur begrenzt notwendig ist. Zielsetzung der intramedullären Bruchstabilisierung unter Frakturkompression ist es, eine verlässliche Stabilisierung der gebrochenen Knochenteile ohne Gefahr von Dislokationen zu erreichen, eine schnelle Knochenbruchheilung aufgrund der Kompression der Knochenteile zu stimulieren und eine Traumatisierung des umliegenden Gewebes möglichst zu vermeiden. Fraktur und Bruch bezeichnen in diesem Sinne denselben Umstand und sind mit übereinstimmendem Sinngehalt und identischem Bedeutungsumfang zu interpretieren. Ein möglichst minimal-invasiver Eingriff fördert dabei eine schnelle Heilung und Rehabilitation und senkt außerdem das postoperative Schmerzniveau des Patienten. Indem das Verbindungsstück zweiteilig ausgeführt ist, wird die Handhabung bei der Implantation vereinfacht, da das Verbindungsstück nicht als Ganzes durch den Bruchspalt in einen Markraum des gebrochene Röhrenknochens eingeschoben werden muss, sondern ein erstes Teil des Verbindungselementes in den Markraum eines ersten Teiles des gebrochenen Röhrenknochens und ein zweites Teil des Verbindungselementes in einen Markraum eines zweiten Teiles des gebrochenen Röhrenknochens eingeschoben werden kann, wodurch die Zugänglichkeitserfordernisse zum Einfügen des Formelementes durch den Bruchspalt in einen Markraum reduziert werden, eine etwaige Markhöhlenpräparation im geringeren Ausmaß notwendig ist und ein Trauma der umliegenden Gewebsstrukturen, insbesondere der Versorgungsstrukturen des Periosts, minimiert wird. Dadurch ist es möglich, bei fachgerechter Implantation, die Rehabilitationszeit und das postoperative Schmerzniveau zu reduzieren und eine rasche Belastungssteigerung in der postoperativen Phase zu ermöglichen. Zudem eröffnet die zweiteilige Ausbildung des Verbindungselementes eine Möglichkeit, insbesondere bei größeren Röhrenknochen, eines oder beide Teile des Verbindungselementes nicht durch den Frakturspalt, sondern etwa durch eine in den Knochen eingebrachte Bohrung bzw. Öffnung hindurch an eine vorgesehene Position an der Bruchstelle einbringen zu können.

Durch das in die Markhöhlen der gebrochenen Röhrenknochen eingesetzte Verbindungselement wird eine mechanische Stabilisierung der Bruchstelle bewirkt, wodurch wirkende Belastungen, beispielsweise durch Translations- oder Biegekräfte, ohne die Gefahr einer Dislokation aufgenommen und abgeleitet werden können. Insbesondere Biegekräfte können hierdurch effektiv neutralisiert werden.

Durch den durch die zumindest eine Durchgangsöffnung des Verbindungselementes durchführbaren Faden können die beiden Röhrenknochenteile in axialer Richtung zugfest aneinander gespannt werden, wodurch auch eine relative Verschiebung der Röhrenknochenteile in axialer Richtung unterbunden wird und zusätzlich durch die aufgebrachte Kompressionskraft an der Bruchfläche eine biologische Knochenheilung stimuliert wird. Der Faden wird dabei durch zumindest ein Ankerelement an zumindest einem Röhrenknochenteil befestigt, wobei unter Ankerelement in einer einfachen Ausführung einfach nur eine Verdickung des Fadens, ein in den Faden eingebrachter Knoten oder ein Fixieren des Fadens durch Klebstoff zu verstehen ist. In anderen Ausführungen kann das zumindest eine Ankerelement aus einem Nippel, einem Schraubelement, einer Ankerplatte oder einer anderen zweckmäßigen endseitigen Fadenbefestigung bestehen. Als besonders zweckmäßig hat sich dabei ein Ankerelement mit einer knopfähnlichen Form erwiesen, da eine solche ein kleines Volumen aufweist und eine Ausbildung eines solchen Ankerelementes in gebogener Form ein Anlegen des Ankerelementes an eine gekrümmte Oberfläche eines Röhrenknochens ermöglicht. Eine weitere Möglichkeit besteht darin, ein Ankerelement als in einen Knochen einschraubbares Element auszubilden. Ein solches einschraubbares Element bietet den Vorteil, dass der Einschraubvorgang des Elementes gleichzeitig zum Spannen eines am Element befestigten Fadens verwendet werden kann. Der am Element befestigte Faden wird durch den Einschraubvorgang um das Element gewickelt und dadurch gespannt. Bevorzugt weist das Element zumindest einen Durchgangskanal auf, durch welchen ein Faden durchführbar ist, um einfach und sicher am Element befestigbar zu sein. Mit Vorteil ist ein solches einschraubbares Element derart ausgeführt, dass dieses, ähnlich einer Senkkopfschraube, nach einem Einschraubvorgang in einen Knochen bündig mit der Knochenoberfläche abschließt. Zweckmäßig kann es jedoch auch sein, ein solches einschraubbares Element ähnlich anderen bekannten Schraubentypen als Flachkopf-, Rundkopf-, Linsenkopf- oder Madenschraube auszuführen.

Es erweist sich als zweckmäßig, wenn zumindest ein Fadenende durch zumindest ein in ein Röhrenknochenteil eingebrachtes Bohrloch vom Markraum des Röhrenknochenteiles nach außen geführt wird und außerhalb der Kortikalis, vorteilhafterweise lösbar, an einem Ankerelement befestigt bzw. gespannt wird. Zweckmäßig kann dabei das Ankerelement als in das Bohrloch einfügbares oder einschraubbares Element ausgeführt sein. Vorteilhaft kann dabei das Ankerelement zumindest einen Durchgangskanal aufweisen, durch welchen der Faden nach außen geführt werden kann. Bevorzugt werden beide Fadenenden durch Bohrlöcher in den beiden Röhrenknochenteilen nach außen geführt und außerhalb der Röhrenknochenteile mit Ankerelementen befestigt bzw. der Faden gespannt.

Der zumindest eine Faden kann dabei einfach oder mehrfach verlegt werden und verläuft vorzugsweise in Knochenachsenrichtung und mittig im Verbindungselement. Der Faden kann dabei auch in das Material des Verbindungselementes eingegossen sein, um eine operative Handhabung zu erleichtern. Zweckmäßig ist der Faden aus einem physiologisch verträglichen Material gebildet.

Besonders bevorzugt ist vorgesehen, dass ein erstes Teil des Verbindungselementes in einem Teil des gebrochenen Röhrenknochens fixierbar ist und ein zweites Teil des Verbindungselementes in das andere Teil des gebrochenen Röhrenknochens einführbar ist. Durch die Fixierung des ersten Teiles des Verbindungselementes in einem Teil des gebrochenen Röhrenknochens wird eine stabile, positionsbeständige Positionierung des Verbindungselementes im Bereich der Bruchstelle sichergestellt. Die Gefahr einer möglichen Verschiebung oder eines Verrutschens des Verbindungselementes ist damit vermieden. Weiter kann ein axiales Spannen der Röhrenknochenteile damit schon dadurch erreicht werden, dass das in einem Röhrenknochenteil fixierte Teil des Verbindungselementes mit dem zumindest einen Faden an das andere Röhrenknochenteil gespannt wird. Zweckmäßig kann ein Ende des zumindest einen Fadens dabei an dem fixierten Verbindungselementteil durch ein Ankerelement befestigt sein, an einem in das fixierte Verbindungselement eingebrachten Bohrloch befestigt sein oder auch im fixierten Verbindungselementteil eingegossen sein. Vorteilhaft muss damit der Faden nur an einem Röhrenknochenteil befestigt werden, womit der operative Aufwand massiv reduziert ist, da beispielsweise etwa nur ein Röhrenknochenteil angebohrt werden muss, um eine Fixierung und ein Spannen des Fadens außerhalb der Kortikalis an einem Ankerelement zu ermöglichen.

Von Vorteil ist es, wenn das erste Teil des Verbindungselementes ein Gewinde aufweist, insbesondere ein selbstschneidendes Gewinde, mit welchem dieses im gebrochenen Röhrenknochen fixierbar ist. Durch ein Gewinde kann das erste Teil des Verbindungselementes einfach und kraftschlüssig an einem Röhrenknochenteil fixiert werden. Der operative Aufwand ist reduziert, da eine aufwendige Markhöhlenpräparation an diesem Röhrenknochenteil entfällt bzw. nur in eingeschränkter Form durchgeführt werden muss.

Zweckmäßig ist es, wenn die beiden Teile des Verbindungselementes durch eine Schraubverbindung oder einen Verschluss, insbesondere einen Schnellverschluss, bevorzugt einen Bajonettverschluss oder einen Schnappverschluss, miteinander verbindbar ausgeführt sind. Allgemein ist eine lösbare Verbindung beliebiger Art ausreichend. Dadurch können die beiden Teile des Verbindungselementes einfach und schnell miteinander verbunden werden und ein stabiles und belastbares Stabilisierungselement im Knochen bilden. Es kann von Vorteil sein, wenn die beiden Teile des Verbindungselementes lösbar verbunden werden, beispielsweise durch einen Bajonettverschluss, eine Schraubverbindung, eine Klemmverbindung oder eine ähnliche zweckmäßige lösbare Verbindungsform. Mit Vorteil kann vorgesehen sein, dass ein zusätzliches Fixierungselement, beispielsweise eine einschraubbare Fixierungsschraube, am Verschluss bzw. der Verbindung vorgesehen ist, um ein ungewolltes Lösen oder eine ungewollte Lockerung des Verschlusses bzw. der Verbindung zu unterbinden.

Vorteilhaft sind die beiden Teile des Verbindungselementes mit einem Bajonettverschluss lösbar verbindbar ausgeführt, um ein einfaches und schnelles Verbinden zu ermöglichen. Hierzu weist ein Teil des Verbindungselementes zumindest einen Verriegelungszapfen auf, welcher normal zur Verbindungsrichtung ausgeformt ist und in zumindest eine korrespondierende Führung am anderen Teil des Verbindungselementes einfügbar ist. Die Führung umfasst dabei eine im Wesentlichen parallel zur Verbindungsrichtung geformte erste längsausgerichtete Ausnehmung, welche in eine im Wesentlichen normal zur Verbindungsrichtung ausgerichtete zweite Ausnehmung übergeht. Damit ist die Führung als L-ähnliche Form ausgeführt und ermöglicht ein Verbinden der beiden Teile des Verbindungselementes durch eine Steck-Dreh-Bewegung. Beim Verbinden wird der Verriegelungszapfen zuerst durch eine translatorische Bewegung entlang der längsgerichteten ersten Ausnehmung geführt und anschließend durch eine rotatorische Bewegung in die im Wesentlichen quer dazu ausgerichtete zweite Ausnehmung weitergeführt, wodurch eine form- bzw. kraftschlüssige Verbindung und Verriegelung des Bajonettverschlusses erfolgt. An die zweite Ausnehmung können zweckmäßig weitere Ausnehmungen anschließen, bevorzugt eine dritte Ausnehmung, welche im Wesentlichen wieder parallel zur Verbindungsrichtung geformt ist. Insbesondere wenn die dritte Ausnehmung im Wesentlichen entgegen der Verbindungsrichtung geformt ist, wird dadurch eine ausgeprägte formschlüssige Fixierung des Verriegelungszapfens erreicht, welche insbesondere bei Zugbelastung ein ungewolltes Öffnen des Bajonettverschlusses verhindert. Mit Vorteil kann die zweite Ausnehmung auch mit einer Steigung in Richtung der Verbindungsrichtung, insbesondere aber auch entgegen der Verbindungsrichtung, ausgeführt sein, wodurch eine ausgeprägte Fixierung des Verriegelungszapfens in der Führung erreichbar ist. Es kann auch zweckmäßig sein die Führung als Nut an der Innenoberfläche eines Teiles des Verbindungselementes auszuführen, wodurch eine Beeinträchtigung der Außenoberfläche des betreffenden Teiles vermieden wird. Eine belastbare Verbindung kann insbesondere dadurch erreicht werden, dass zwei oder mehr Verriegelungszapfen vorgesehen sind, welche an gegenüberliegenden Seiten eines Teiles des Verbindungselementes ausgeformt sind. Bevorzugt kann auch vorgesehen sein, dass der zumindest eine Verriegelungszapfen in einer Verschlussposition in der Führung federbelastet ist, wodurch eine ausgeprägte kraftschlüssige Verbindung erreicht werden kann.

Als besonders effizient kann auch ein Schnappverschluss gesehen werden. Ein solcher, insbesondere in einer einfachen nicht lösbaren Ausführung, erfordert kaum operativen Aufwand und kann insbesondere bei eingeschränkter Zugänglichkeit zur Frakturstelle effektiv verwendet werden.

Von Vorteil ist es, wenn zumindest ein Abschnitt des Verbindungselementes mit einer Mehrkantoberfläche ausgebildet ist, um ein Eindrehen und/oder Einschrauben des Verbindungselementes bzw. eines Teiles des Verbindungselementes mit einem Werkzeug, insbesondere einem Gabelschlüssel, zu ermöglichen. Indem ein oder mehrere Abschnitte des Verbindungselementes, insbesondere zumindest ein Abschnitt am ersten und/oder zweiten Teil des Verbindungselementes, mit einer Mehrkantoberfläche ausgebildet sind, ist ein leichtes Handhaben und Einbringen des Verbindungselementes bzw. eines Teiles desselben in den Röhrenknochen ermöglicht. Insbesondere eine Ausbildung mit Vierkant- oder Sechskantprofil hat sich als sehr effizient erwiesen. In einer bevorzugten Variante ist das Mehrkantprofil als versenkte Struktur in die Oberfläche des Verbindungselementes eingebracht, indem in die Oberfläche entlang des Umfanges eines Abschnitts des Verbindungselementes mehrere tangentiale Schnittflächen eingebracht sind. Dadurch fügt sich die Mehrkantstruktur ohne Änderung der Grundform des Verbindungselementes in die Oberfläche ein und stellt kein Hindernis für ein Einfügen des Verbindungselementes in einen Röhrenknochenteil dar.

Es erweist sich als zweckmäßig, wenn das Verbindungselement länglich, insbesondere im Wesentlichen zylinderförmig oder spindelförmig, geformt ist. Damit ist das Verbindungselement an der Grundform eines Röhrenknochens orientiert und ermöglicht eine Stabilisierung der Knochenstruktur entlang eines ausgedehnten Bereiches, um auftretende Kräfte effektiv weiterzuleiten. Um ein Einführen des Verbindungselementes bzw. eines Teiles des Verbindungselementes in einen Markraum zu erleichtern, kann ein Ende eines solchen mit abgerundeten oder konisch verlaufenden Enden geformt sein. Bevorzugt kann das Verbindungselement im Wesentlichen spindelförmig ausgebildet sein, wodurch einerseits ein erleichtertes Einfügen in die Röhrenknochenteile erreicht ist, andererseits aber auch die Bruchstelle verstärkt stabilisiert wird. Grundsätzlich kann das Verbindungselement zweckangepasst von einer zylindrischen Grundform abweichen und insbesondere der Knochengrundform angepasst werden. So kann es sich als vorteilhaft erweisen, ein Verbindungselement mit nicht kreisförmigem Querschnitt auszubilden, insbesondere wenn dieses in Röhrenknochen mit nicht kreisförmigem Querschnitt eingesetzt werden soll. Von Vorteil kann es auch sein, das Verbindungselement an einen Hohlraum des Röhrenknochens, insbesondere an einen Hohlraum an der Bruchstelle, anzupassen, um damit eine besonders ausgeprägte Stabilisierung der Bruchstelle sicher zu stellen.

Grundsätzlich kann das Verbindungselement je nach Anforderungen als Massivelement oder als Hohlkörper ausgebildet sein. Größe, Ausdehnung und/oder spezielle Ausführung der Form des Verbindungselementes orientieren sich an der jeweiligen Größe und/oder Form des betreffenden Röhrenknochens und stellen außerdem eine Abwägung zwischen Gewicht und Stabilität bzw. Volumen und Stabilität des einzubringenden Verbindungselementes dar.

Mit Vorteil kann das Verbindungselement zumindest bereichsweise eine profilierte Oberfläche aufweisen. Indem die Oberfläche zumindest eines Teiles eines Verbindungselementes zumindest bereichsweise eine Profilierung aufweist, wird die Formschlüssigkeit zwischen dem Verbindungselement und der Innenseite des Röhrenknochens verstärkt und das Verbindungselement drehfest im Röhrenknochen positioniert. Die vergrößerte Oberfläche einer profilierten Struktur führt zu einer Verankerung des Verbindungselementes im Röhrenknochen, welche mit dem Heilungsprozess weiter zunimmt, da neu gebildete Knochenstruktur mit der profilierten Oberflächenstruktur eine Verzahnung bildet, welche ein effektives Aufnehmen von wirkenden Torsionskräften, insbesondere im Bereich der Bruchstelle ermöglicht.

Besonders bevorzugt erweist es sich, wenn das Verbindungselement zumindest teilweise mit Längsrippen versehen ist. Indem die Oberfläche zumindest eines Teiles eines Verbindungselementes zumindest bereichsweise mit Längsrippen versehen ist, kann das Verbindungselement besonders drehfest im Röhrenknochen positioniert werden. Auf die Knochenstruktur wirkende Torsionskräfte können damit effektiv aufgenommen und weitergeleitet werden. In einer zweckmäßigen Variante können die Längsrippen dabei im Querschnitt eine rechteckige bzw. rechteckähnliche Form aufweisen. Mit Vorteil können die Längsrippen im Querschnitt eine nach außen spitz zulaufende oder trapezförmige Form aufweisen, wodurch eine ausgeprägte Verzahnung mit der Röhrenknocheninnenstruktur erreichbar ist.

Abhängig von Anwendungsfall und Knochenform kann die zumindest eine Durchgangsöffnung durch beide oder nur durch eines der Teile des Verbindungselementes verlaufen, um eine zweckangepasste Durchführung des Fadens durch das Verbindungselement zu ermöglichen und ein beständiges Spannen der Röhrenknochenteile sicherzustellen. Vorteilhaft ist es, wenn sich die zumindest eine Durchgangsöffnung des Verbindungselementes in Längsrichtung des Verbindungselementes durch das gesamte Verbindungselement erstreckt, um eine Durchführung des Fadens durch das gesamte Verbindungselement zu ermöglichen. Dadurch stellt das Verbindungselement eine Führung für den durchgeführten Faden dar und ermöglicht ein hindernisfreies und zugfestes Spannen der Röhrenknochenteile. Insbesondere wenn das Verbindungselement als Massivelement ausgebildet ist, kann die zumindest eine Durchgangsöffnung durch zumindest ein in axialer Richtung in das Verbindungselement eingebrachtes Bohrloch gebildet sein. Ein solches Bohrloch stellt eine Durchgangsöffnung mit im Wesentlichen konstantem Innendurchmesser dar und verläuft bevorzugt zentral in axialer Richtung durch das Verbindungselement. Dadurch wird eine Führung des Fadens im Wesentlichen zentral in axialer Richtung durch den Röhrenknochen sichergestellt und ermöglicht ein nachhaltiges und ungehindertes Spannen der Röhrenknochenteile.

Es kann auch vorgesehen sein, dass mehrere Durchgangsöffnungen bzw. Bohrlöcher in das Verbindungselement eingebracht sind, um eine mehrfache Durchführung zumindest eines Fadens zu ermöglichen. Dadurch kann eine besonders nachhaltige und ausgeprägte Zugspannung umgesetzt werden, insbesondere wenn hohe spätere Belastungen zu erwarten sind.

Von Vorteil ist es, wenn das Verbindungselement, insbesondere zumindest eines der Teile des Verbindungselementes, zumindest eine Führungsrille aufweist, durch welche der zumindest eine Faden führbar ist, um das Verbindungselement bzw. zumindest eines der Teile des Verbindungselementes rotationsfest im Röhrenknochen zu positionieren. Indem beim Spannen des zumindest einen Fadens der Faden durch die zumindest eine Führungsrille geführt ist, wird eine Relativposition des Verbindungselementes bzw. eines Teiles des Verbindungselementes im Röhrenknochen gegen Rotation festgelegt und ein ungewolltes Rotieren des Verbindungselementes bzw. eines Teiles des Verbindungselementes um dessen Längsachse verhindert. Die Führungsrille kann dabei zweckmäßig als Einkerbung, Inzisur, Nut, Rille oder jegliche andere Form eine Ausnehmung oder Vertiefung, welche geeignet ist den Faden zu führen, ausgebildet sein.

Bevorzugt ist vorgesehen, dass die zumindest eine Führungsrille in einem Bereich des Führungselementes angeordnet ist, an welcher der zumindest eine Faden aus dem Verbindungselement austritt, wodurch eine beständige Führung des Fadens erreicht wird. Insbesondere an einem der Enden des Führungselementes bzw. eines dessen Teile kann eine Führungsrille besonders einfach, etwa durch eine Kerbe oder einen Einschnitt, eingebracht werden, wodurch der zumindest eine Faden besonders robust geführt werden kann. Eine besonders beständige Führung des Fadens wird erreicht, wenn die Führungsrille als Nut oder Rille ausgebildet ist, welche sich entlang einer Innenoberfläche der zumindest einen Durchgangsöffnung erstreckt.

Zweckmäßig kann es sein, wenn das Verbindungselement, insbesondere eines oder beide Teile des Verbindungselementes, als Hohlstruktur, insbesondere als Hohlzylinder, ausgebildet sind. Dadurch kann das Verbindungselement sowohl der Stabilisierungsfunktion als auch einem Erfordernis der Gewichtsreduktion bzw. Volumenreduktion gerecht werden. Auch stellt ein in ein biologisches System eingebrachtes Implantat, insbesondere abhängig von den für das Implantat verwendeten Materialien, einen Fremdkörper dar, weshalb eine eingebrachte Fremdmaterialmenge grundsätzlich möglichst klein gehalten werden sollte, um mögliche negative Reaktionen, insbesondere Abstoßungs- und Entzündungsreaktionen, zu vermeiden. Vorteilhaft kann die Hohlstruktur an der Form des zu behandelnden Röhrenknochens orientiert sein. Auch kann es vorteilhaft sein, das Verbindungselement an einen Hohlraum des Röhrenknochens anzupassen, insbesondere einen Hohlraum des Röhrenknochens an der Bruchstelle, um damit eine besonders ausgeprägte Stabilisierung der Bruchstelle zu erreichen.

In einer weiteren vorteilhaften Ausprägung kann das Verbindungselement als Hohlkammerstruktur ausgebildet sein. Dadurch wird ein Kompromiss zwischen Stabilisierungsfunktion und Gewichtsreduktion bzw. Volumenreduktion erreicht. In einer besonderen Ausprägung kann dabei das Verbindungselement eine Hohlstruktur, insbesondere eine im Wesentlichen hohlzylindrische Struktur aufweisen, deren Hohlraum mit Stützelementen, beispielsweise Stützstreben, ausgebildet ist, wodurch ein effektives Ableiten von auf die Hohlstruktur wirkenden Kräften erreichbar ist. Vorteilhaft kann mit einer solchen Struktur außerdem Einfluss auf die Biegesteifigkeit des Verbindungselementes genommen werden, wodurch dieses an die Elastizitäts- und Steifigkeitseigenschaften eines Röhrenknochens angepasst werden kann.

Von Vorteil ist es auch, wenn die Enden der beiden Teile des Verbindungselementes verjüngt oder abgeschrägt ausgebildet sind. Dadurch ist ein einfaches Einfügen der Verbindungselementteile in die Röhrenknochenteile gewährleistet. Vorteilhaft ist hierbei außerdem, wenn die Enden der Verbindungselementteile abgerundet ausgebildet sind, wodurch ein Einfügen der Verbindungselementteile in den Röhrenknochen möglichst gleitend durchgeführt werden kann.

In einer besonderen Variante ist es zweckmäßig, wenn die beiden Verbindungselementteile durch ein Stecken formschlüssig verbindbar sind und das Verbindungselement zumindest zwei Durchgangsöffnungen aufweist, welche sich in Längsrichtung des Verbindungselementes, insbesondere im Wesentlichen parallel, durch das gesamte Implantat erstrecken, wodurch mit einer Durchführung eines Fadens durch die beiden Durchgangsöffnungen die beiden Teile des Verbindungselementes aneinander anspannbar sind. Indem die beiden Verbindungselementteile durch ein Stecken formschlüssig verbindbar sind, wird eine stabile Verbindung zumindest in zwei Dimensionen erreicht. Um außerdem ein Verbinden der beiden Verbindungselementteile in axialer Richtung sicherzustellen, kann zumindest ein Faden derart durch die zumindest zwei Durchgangsöffnungen durchgeführt werden, dass die beiden Verbindungselementteile aneinander angespannt sind. Dabei kann zum Spannen der beiden Röhrenknochenteile und zum Aneinanderspannen der beiden Verbindungselementteile derselbe Faden verwendet werden. Es ist aber auch möglich, mehrere Fäden zu verwenden, um die beiden Spannvorgänge strukturell voneinander zu trennen. In einer Ausführungsform, bei welcher vorgesehen ist, dass das erste Teil des Verbindungselementes in einem Teil des Röhrenknochens fixiert ist, kann besonders effizient das Aneinanderspannen der beiden Teile des Verbindungselementes und das Spannen des fixierten Teiles des Verbindungselementes an das andere Röhrenknochenteil mit nur einem Faden durchgeführt werden. Hierzu wird der Faden derart durch die zumindest zwei Durchgangsöffnungen der beiden Teile des Verbindungselementes durchgeführt, dass beide Fadenenden auf jener Seite zu liegen kommen, von welcher aus die beiden Knochenteile aneinander gespannt werden können. Der Faden verläuft dabei also durch eine erste Durchgangsöffnung hindurch und durch zumindest eine zweite Durchgangsöffnung wieder zur Ausgangsseite zurück. Wird nun ein Ende des Fadens fixiert, beispielsweise mit einem Ankerelement, können mit dem zweiten Ende des Fadens sowohl die beiden Verbindungselementteile aneinander als auch das im ersten Teil des Röhrenknochens fixierte Verbindungselementteil an das zweite Teil des Röhrenknochens gespannt werden. Eine Ausführungsvariante mit Steckverbindung, welche ein Aneinanderspannen der beiden Teile des Verbindungselementes mit einem Faden vorsieht, birgt den Vorteil einer einfachen Ausgestaltung des Verbindungselementes, da auf einen Verschluss zum Verbinden der beiden Teile des Verbindungselementes verzichtet werden kann.

Vorteilhaft kann es sein, wenn ein Teil des Verbindungselementes als Spreizdübel ausgeführt ist. Durch ein Verspreizen eines Teiles des Verbindungselementes in einem Röhrenknochenteil kann ein ungewolltes Verrutschen des Verbindungselementes verhindert werden. Abhängig vom für ein Teil des Verbindungselementes verwendeten Material kann ein Verspreizen durch plastische Verformung eines Bereiches des Verbindungselementteiles und/oder durch mechanische Verklemmung, beispielsweise durch eine widerhakenähnliche Ausprägung, erfolgen, um damit eine form- und/oder kraftschlüssige Fixierung des Verbindungselementes bzw. eines Teiles davon sicherzustellen. Besonders vorteilhaft kann es sein, wenn ein erstes Teil des Verbindungselementes sowohl ein Gewinde, insbesondere ein selbstschneidendes Gewinde, aufweist als auch als Spreizdübel ausgeführt ist. Durch diese Kombination von zwei unterschiedlichen Fixierungsvarianten kann eine besonders nachhaltige und sichere Fixierung des Verbindungselementes im Röhrenknochen sichergestellt werden.

Zweckmäßig kann es sein, wenn der Spreizdübel derart ausgebildet ist, dass dieser sich erst durch das Verbinden mit dem anderen Teil des Verbindungselementes, insbesondere durch eine Schraubverbindung, aufspreizt. Damit ist eine Positionierung des Verbindungselementes im Röhrenknochen erleichtert, da die Fixierungsposition gleichzeitig mit dem Verbinden der beiden Teile des Verbindungselementes festgelegt wird.

Es kann auch vorgesehen sein, dass ein Teil des Verbindungselementes teleskopartig ausgebildet ist und beim Aufbringen einer Zugkraft auf das Implantat ausfahrbar ist. Eine solche Ausführungsvariante stellt eine besonders platzsparende Variante dar und ermöglicht ein leichtes Einfügen eines Teiles des Verbindungselementes in ein Teil des Röhrenknochens durch einen Frakturspalt hindurch. Indem eine solche Teleskopstruktur erst beim Wirken einer Zugkraft, insbesondere durch einen an ein Teil eines Verbindungselementes befestigten Faden, ausgefahren wird, ist eine einfache und handlungsoptimierte Implantation verwirklicht. Im Speziellen kann damit das Spannen der beiden Röhrenknochenteile und das Ausfahren der Teleskopstruktur in einem gemeinsamen Schritt erfolgen.

Besonders bevorzugt ist es, wenn ein erstes Teil des Verbindungselementes in einem ersten Teil des Röhrenknochens fixierbar ist, insbesondere durch ein Gewinde, zum Beispiel ein selbstschneidendes Gewinde, und das zweite Teil des Verbindungselementes teleskopartig ausgebildet ist. Ist der Faden zum Spannen der beiden Röhrenknochenteile am zweiten Teil des Verbindungselementes befestigt, kann nach einem Verbinden der beiden Teile des Verbindungselementes ein Ausfahren der teleskopartigen Struktur sowie das Spannen der Röhrenknochenteile in einem Schritt einfach durch ein Spannen und zugfestes Befestigen des Faden am zweiten Teil des Röhrenknochens erfolgen.

Der zumindest eine Faden kann über zumindest eine Durchgangsöffnung durch das Verbindungselement durchführbar sein, um die beiden Röhrenknochenteile zugfest zu spannen. Es ist aber auch möglich den zumindest einen Faden an zumindest einem Teil des Verbindungselementes zu befestigen bzw. zu fixieren, beispielsweise indem der Faden in zumindest ein Teil des Verbindungselementes eingegossen ist oder das Verbindungselement bzw. ein Teil davon eine Durchgangsbohrung aufweist, um den Faden daran zu fixieren. Es kann von Vorteil sein, wenn der zumindest eine Faden am Verbindungselement bzw. einem Teil des Verbindungselementes vormontiert ist, um den operativen Aufwand zu reduzieren. Der zumindest eine Faden kann über zumindest ein Ankerelement am Verbindungselement bzw. einem Teil davon befestigbar sein. Insbesondere wenn ein Teil des Verbindungselementes in einem ersten Teil des Röhrenknochens fixierbar ist, kann ein Spannen der beiden Röhrenknochenteile dadurch erreicht werden, dass das zweite Teil des Röhrenknochens mit dem zumindest einen Faden an das Verbindungselement bzw. an ein Teil des Verbindungselementes gespannt wird.

Mit Vorteil sind Teile des Implantats, insbesondere das Verbindungselement, zumindest teilweise aus Stahl, einer Titan-Legierung, einer Magnesium-Legierung oder einer Keramik gebildet, um eine hohe Robustheit zu erreichen. Im Speziellen die Verwendung von Ti-Al-V Legierungen, insbesondere Ti-6Al-4V, hat sich für Implantate aufgrund deren Biokompatibilität als zweckmäßig erwiesen. Abhängig vom zu behandelnden Röhrenknochen und den zu erwartenden Belastungen kann auch eine Kombination von verschiedenen Materialien zweckmäßig sein, um die Stabilisierungsfunktion des Implantates zu optimieren.

Bevorzugt ist es, wenn der zumindest eine Faden zumindest teilweise aus Stahl oder einer Titan-Legierung gebildet ist. Da der Faden zum Spannen der beiden Röhrenknochenteile verwendet wird, muss dieser geeignet sein einer auf den Knochen wirkenden Zugbelastung, insbesondere bei postoperativer Beanspruchung, zu widerstehen. Gleichzeitig sollte der Faden bevorzugt aus einem physiologisch verträglichen Material gebildet sein. Unter Berücksichtigung der jeweiligen Belastungsanforderungen kann der Faden aus einem der üblichen verfügbaren chirurgischen Nahtmaterialien gebildet sein. Um die Reißfestigkeit zu erhöhen kann der Faden auch aus mehreren Einzelfasern geflochten und/oder mehrfach verlegt sein.

Eine hohe Biokompatibilität wird erreicht, wenn das Verbindungselement und/oder das zumindest eine Ankerelement und/oder der zumindest eine Faden zumindest teilweise aus Polyetheretherketon, auch bezeichnet als PEEK, gebildet sind. Zudem weist dieses Material eine hohe Robustheit und Hydrolysebeständigkeit auf.

Vorteilhaft ist es, dass das Verbindungselement und/oder das zumindest eine Ankerelement und/oder der zumindest eine Faden zumindest teilweise aus resorbierbarem Material gebildet sind. Dies bietet den Vorteil, dass ein eingesetztes Implantat bzw. zumindest Teile davon nicht im Rahmen einer Zweit-Operation wieder entfernt werden müssen, da diese mit der Zeit degradieren und/oder vom biologischen System abgebaut bzw. resorbiert werden. Während Ankerelemente und Faden relativ leicht wieder entfernt werden können, kann dadurch insbesondere eine aufwendige Entfernung des Verbindungselementes vermieden werden. Der zumindest eine Faden kann dabei etwa mit mehreren, insbesondere verdrehten oder versponnenen, Fasern gebildet sein. Indem ein Faden sowohl Fasern aus resorbierbarem Material als auch Fasern aus nicht resorbierbarem Material aufweist, kann ein Faden teilweise resorbierbar ausgebildet sein, und damit ein Kompromiss zwischen Belastbarkeit und Resorbierbarkeit erreicht werden. Eine hohe Zugfestigkeit des Fadens ist insbesondere dadurch erreichbar, dass ein Faden aus mehreren miteinander verdrehten bzw. verdrillten Fäden gebildet ist.

Ein weiterer Vorteil ist dadurch gegeben, dass eine Degradationsgeschwindigkeit von Implantatteilen, insbesondere des Verbindungselementes, auf den Heilungsprozess abgestimmt werden kann. Der Knochen erhält durch Heilung sukzessive seine Eigenstabilität und Belastbarkeit zurück und im selben Maße kann die Stützfunktion der Implantatteile reduziert werden. Die Degradationsgeschwindigkeit kann für verschiedene Abschnitte von Implantatteilen, insbesondere für die Teile des Verbindungselementes bzw. Abschnitte davon, unterschiedlich eingestellt werden. Weiter kann diese außerdem auf das Stoffwechselsystem des umgebenden biologischen Systems abgestimmt werden, um den Heilungsprozess zu optimieren.

Bevorzugt ist das Verbindungselement und/oder das zumindest eine Ankerelement und/oder der zumindest eine Faden zumindest teilweise aus Polymilchsäure oder einem Derivat davon gebildet sind. Eine besondere Bedeutung bei resorbierbaren Materialien kommt der Biokompatibilität und der Gewebeverträglichkeit der verwendeten Materialkomponenten zu. Insbesondere sollten Materialkomponenten bzw. Materialelemente vermieden werden, welche Allergien oder Entzündungen auslösen können. Aus resorbierbaren Materialien gefertigte Implantatteile, insbesondere Teile des Verbindungselementes, sollten daher vorteilhaft aus Materialien gebildet sein, deren Komponenten bzw. Elementbestandteile vollständig biologisch abbaubar sind. Zweckmäßig können hierzu Materialien aus Polymilchsäure oder einem Derivat davon gebildet sein. Insbesondere Poly-L-Lactid, auch bezeichnet als PLLA, hat sich aufgrund dessen ausgeprägten mechanischen Festigkeit als besonders geeignet herausgestellt. Die Degradationsgeschwindigkeit kann vorteilhaft über die genaue chemische Zusammensetzung, Porosität und/oder Kristallinität des eingesetzten Materials beeinflusst werden.

Ein Nachteil von biodegradierbaren Polymerstrukturen ist eine im Vergleich zu Metallen geringere mechanische Festigkeit. Weiter kommt es beim Abbauprozess von Polymerstrukturen häufig zu einer Volumenzunahme der Implantatstrukturen, da der Abbauprozess unter Hydrolyse stattfindet. Es kann daher vorteilhaft sein Implantatteile aus biodegradierbaren Metalllegierungen zu bilden, welche sich insbesondere durch eine höhere Festigkeit auszeichnen. Festgestellt wurde, dass für die Bildung von intramedullären Implantatteilen die Verwendung einer Mg-Zn-Ca-Legierung besonders geeignet ist.

Erfindungsgemäß ist vorgesehen, dass das Verbindungselement und/oder das zumindest eine Ankerelement und/oder der zumindest eine Faden zumindest teilweise aus einer Magnesiumbasislegierung gebildet sind, wobei die Legierung (in Gew.-%) enthält:
mehr als 0,0 bis 5,0 % Zn,
mehr als 0,0 bis 1,0 % Ca,
optional mehr als 0,0 bis 2,0 % Mn,
Rest Mg und herstellungsbedingte Verunreinigungen.

Diese Legierung enthält oder besteht im Wesentlichen aus Elementen, welche in menschlichen und tierischen biologischen Systemen bereits vorhanden sind, weshalb diese eine ausgeprägte Biokompatibilität aufweisen und Reiz- und Reaktionserscheinungen vermieden werden. Die Legierung weist ausgezeichnete Degradierungs- bzw. Resorptionseigenschaften auf und kann praktisch vollständig von einem menschlichen oder tierischen biologischen System abgebaut werden. Vorteilhaft kann außerdem die Festigkeit und Degradationsgeschwindigkeit über die Zusammensetzung, Porosität und/oder Bildung von intermetallischen Phasen im Material eingestellt werden. Zweckmäßig kann durch eine abgestimmte Wärmebehandlung der Legierung der Anteil von verschiedenen intermetallischen Phasen in der Magnesiummatrix bzw. im Magnesiumrest gezielt eingestellt und damit auf das Degradationsverhalten Einfluss genommen werden. Insbesondere durch ein abgestimmtes Verhältnis von Mg₂Ca- und Ca₂Mg₆Zn₃-Phasen kann die Degradationsrate vorherbestimmt werden. Eine Verwendung einer solchen Legierung für Teile des intramedullären Implantats bietet damit einerseits den Vorteil einer vollständigen Resorbierbarkeit und andererseits, da es sich um ein metallisches Material handelt, außerdem eine erhöhte Festigkeit, insbesondere eine Zugfestigkeit von etwa 250 MPa, wodurch die Stabilität von behandelten Röhrenknochen massiv gesteigert werden kann.

Für eine hohe Festigkeit vorteilhaft hat sich ein Zn-Anteil kleiner als 2,0 Gew.-%, besonders bevorzugt kleiner als 1,0 Gew.-%, erwiesen. Besonders vorteilhaft hinsichtlich Degradationsrate und Biokompatibilität ist es, wenn ein Zn-Anteil kleiner als 1,0 Gew.-% und ein Ca-Anteil kleiner als 1,0 Gew.-% sind. Eine zusätzliche Aufnahme von Zn und/oder Ca durch die Degradierung von Implantatteilen ist damit völlig unbedenklich. Die Möglichkeit die Degradationsgeschwindigkeit genau einzustellen ergibt darüber hinaus einen weiteren Vorteil. Der hydrolytische Abbauprozess von bioresorbierbaren Materialien, ist üblicherweise mit sauren Degradationsprodukten, insbesondere einer hohen Wasserstoffkonzentration, verbunden, welche lokal einen sehr niedrigen pH-Wert bewirken können. Eine auf die Stoffwechselumgebung abgestimmte Degradationsgeschwindigkeit ermöglicht es, eine zu hohe Wasserstoffkonzentration zu vermeiden.

Weiter konnte gezeigt werden, dass durch den Einsatz eines Equal-channel-angular-pressing-Verfahrens bei der Herstellung der Implantatteile aus einer solchen Legierung, die Belastbarkeit maßgeblich weiter erhöht werden kann und die Festigkeit um bis zu 60 % erhöht werden kann. Durch das Pressen des Materials durch eine Matrize mit zwei Kanälen, welche einen definierten Winkel zueinander aufweisen, kann Einfluss auf die Homogenität und Feinkörnigkeit der Mikrostruktur des Materials genommen werden und insbesondere dessen Festigkeit und Härte erhöht werden. Als zweckmäßig hat sich dabei eine Umformung in einem Winkelbereich von 90° bis 130° erwiesen. Vorteilhaft für die Feinkörnigkeit der Mikrostruktur kann dabei ein Winkel zwischen 105° und 115°, besonders vorteilhaft von etwa 110°, sein. Insbesondere durch mehrfaches Umformen kann die Festigkeit, die Dauerwechselfestigkeit und das Ermüdungsverhalten des Materials weiter verbessert werden. In einer speziellen Variante kann ein Mehrfachumformen dabei auch dadurch erfolgen, dass das Material durch eine Matrize mit drei Kanälen, welche die genannten Winkelbereiche zueinander aufweisen, gepresst wird. Dadurch ist es möglich die Festigkeit eines Implantatteiles, insbesondere des Verbindungselementes bzw. eines Teiles davon, auf eine spätere Belastung abzustimmen. Eine zumindest teilweise Bildung von Implantatteilen aus diesem Legierungsmaterial ermöglicht damit einen Einsatz in besonders belasteten Röhrenknochensystemen, ohne auf den Vorteil der Resorbierbarkeit, insbesondere ohne Nebenwirkungen, verzichten zu müssen. Damit ist es möglich kleinere Implantate bzw. Implantatteile herzustellen, welche auch für eine Behandlung von dünneren Röhrenknochen, insbesondere Röhrenknochen mit einem dünnen Markraum, beispielsweise Mittelhandknochen oder Rippen, geeignet sind.

Entsprechend bietet es auch Vorteile, eine solche Legierung auch für ein einteilig ausgebildetes Implantat zu verwenden. Es wird daher gemäß der Erfindung vorgeschlagen, ein Implantat zum zugfesten Verbinden zweier Teile eines gebrochenen Röhrenknochens gemäß einem vorstehend dargestellten Aufbau mit einem einteilig ausgebildeten Verbindungselement auszubilden, welches aus einer resorbierbaren Magnesiumbasislegierung gebildet ist, wobei die Legierung (in Gew.-%) enthält:
mehr als 0,0 bis 5,0 % Zn,
mehr als 0,0 bis 1,0 % Ca,
optional mehr als 0,0 bis 2,0 % Mn,
Rest Mg und herstellungsbedingte Verunreinigungen.

Ungeachtet der Vorteile einer zweiteiligen Verbindungselementvariante, kann bei leicht zugänglichen Brüchen mit großen Frakturspalten ein einteiliges Verbindungselement praktikabel sein. Eine Ausbildung eines solchen aus einer vollständig resorbierbaren Metalllegierung ermöglicht es, die Stabilitätsfunktion im Vergleich zu beispielsweise im Stand der Technik aus Polymilchsäure gebildeten einteiligen Verbindungselementen massiv zu erhöhen und damit einen verlässlichen Einsatz in besonders belasteten Röhrenknochen sicherzustellen.

Von Vorteil kann es auch sein, wenn das Verbindungselement und/oder das zumindest eine Ankerelement aus porösem Material gebildet sind. Dadurch kann neben einer Reduzierung des Gewichtes bzw. Volumens auch ein etwaiges Degradierungsverhalten des Materials beeinflusst werden. Vorteilhaft kann weiter sein, dass eine poröse Materialstruktur wirkungsaktive Substanzen, beispielsweise Antibiotika und/oder osteoinduktive Subtanzen und/oder Zellen, aufnehmen kann, welche damit bei der Implantation automatisch mit zugeführt werden können. Insbesondere bei der Verwendung eines resorbierbaren porösen Materials ist es damit möglich wirkungsaktive Substanzen über einen längeren Zeitraum verteilt dem biologischen System zuzuführen. Zweckmäßig kann es sein, wenn einzelne Abschnitte ein resorbierbares Material mit unterschiedlichen Degradierungsgeschwindigkeiten aufweisen. Damit kann der Materialabbau optimiert an das Knochenwachstum angepasst werden, ohne dieses zu hemmen, dabei jedoch stets die notwendige Stabilisierungsfunktion aufrechtzuerhalten. Beispielsweise kann das Verbindungselement aus Schichten bestehen, welche unterschiedliche Degradierungsgeschwindigkeiten aufweisen, wodurch der Auflösvorgang des Verbindungselementes gezielt vorgegeben werden kann, eine noch notwendige Stützfunktion jedoch erhalten bleibt.

Eine weitere Variante eines zweiteiligen Implantates, mit den vorgenannten Vorteilen, sieht vor, dass beide Teile eines zweiteiligen Implantates unmittelbar in den Röhrenknochenteilen fixiert werden. Ein solches Implantat kann auch ohne Faden, ohne Ankerelement und ohne Durchgangsöffnung ausgebildet sein, um diese Vorteile zu erreichen. Es wird daher gemäß der Erfindung ein Implantat zum zugfesten Verbinden zumindest zweier Teile eines gebrochenen Röhrenknochens vorgeschlagen, umfassend ein Verbindungselement, wobei das Verbindungselement derart ausgeführt ist, dass dieses in Längsrichtung des gebrochenen Röhrenknochens in einen Markraum des gebrochenen Röhrenknochens einführbar ist, wobei das Verbindungselement zweitteilig ausgeführt ist, wobei ein erstes Teil des Verbindungselementes in einem Teil des gebrochenen Röhrenknochens unmittelbar fixierbar ist und ein zweites Teil des Verbindungselementes im anderen Teil des gebrochenen Röhrenknochens unmittelbar fixierbar ist, wobei das erste Teil mit dem zweiten Teil zur Heilung des gebrochenen Röhrenknochens verbindbar ist. Ein derartig ausgebildetes Implantat ermöglicht auf einfache und praktikable Weise ein zugfestes Verbinden und Stabilisieren der gebrochenen Knochenteile.

Das Verbindungselement bzw. die beiden Teile des Verbindungselementes sind dabei entsprechend den in dieser Anmeldung dargestellten Varianten, Ausprägungen sowie Materialzusammensetzungen und mit den entsprechenden vorteilhaften Wirkungen ausgebildet.

Insbesondere ist dabei auch zweckmäßig, wenn die beiden Teile des Implantates Gewinde, insbesondere selbstschneidende Gewinde, aufweisen, durch welche diese in den gebrochenen Röhrenknochen unmittelbar fixierbar sind. Dadurch können das erste Teil und zweite Teil des Verbindungselementes einfach und kraftschlüssig am jeweiligen Röhrenknochenteil fixiert werden. Der operative Aufwand ist reduziert, da eine aufwendige Markhöhlenpräparation an den beiden Röhrenknochenteilen entfällt bzw. nur in eingeschränkter Form durchgeführt werden muss.

Insbesondere vorteilhaft ist auch, wenn die beiden Teile des Verbindungselementes durch einen Verschluss, insbesondere einen Schnellverschluss, bevorzugt einen Schnappverschluss, miteinander verbindbar ausgeführt sind. Dadurch können die beiden Teile des Verbindungselementes einfach und schnell miteinander verbunden werden und ein stabiles und belastbares Stabilisierungselement im Knochen bilden.

Auch wenn dieses Implantat ohne Faden, Anker und Durchgangsöffnung auskommt, ist es günstig, dieses trotzdem mit Faden, Anker und Durchgangsöffnung entsprechend den erfindungsgemäßen Ausführungen in dieser Anmeldung auszubilden. Dadurch ist die Bruchstabilisierung besonders robust ausgeführt und insbesondere gegen Zugbelastung weiter verstärkt. Zudem kann dadurch eine Kompression der Frakturteile aneinander weiter erhöht und damit eine Knochenheilung effektiver stimuliert werden.

Das weitere Ziel der Erfindung wird durch ein Verfahren der eingangs genannten Art erreicht, bei dem zur Herstellung eines Implantats bzw. eines Teiles davon ein Equal-channel-angular-pressing-Verfahren angewendet wird. Dabei wird das Material durch eine Matrize mit zwei Kanälen, welche einen definierten Winkel zueinander aufweisen, gepresst. Dadurch kann die Homogenität und Feinkörnigkeit der Mikrostruktur des Materials beeinflusst werden und insbesondere dessen Festigkeit und Härte erhöht werden. Als zweckmäßig hat sich dabei eine Umformung in einem Winkelbereich von 90° bis 130° erwiesen. Vorteilhaft für die Feinkörnigkeit der Mikrostruktur kann dabei ein Winkel zwischen 105° und 115°, besonders vorteilhaft von etwa 110°, sein. Insbesondere durch mehrfaches Umformen kann die Festigkeit, die Dauerwechselfestigkeit und das Ermüdungsverhalten des Materials weiter verbessert werden. In einer speziellen Variante kann ein Mehrfachumformen dabei auch dadurch erfolgen, dass das Material durch eine Matrize mit drei Kanälen, welche die genannten Winkelbereiche zueinander aufweisen, gepresst wird.

Durch eine hochgradige plastische Umformung können die Materialeigenschaften von für Implantatteile verwendeten metallischen Materialien an den späteren Anwendungszweck angepasst werden. Insbesondere das Verbindungselement des Implantats muss verlässlich eine Stabilisierung des gebrochenen Röhrenknochens über einen längeren Zeitraum unter variierenden Belastungen gewährleisten können. Durch eine Vorbehandlung des Materials mit einem Equal-channel-angular-pressing-Verfahren, insbesondere in Kombination mit Wärmebehandlungen, können Materialzustände hoher Festigkeit mit verbessertem Ermüdungsverhalten hergestellt werden. Für besonders widerstandsfähige Verbindungselemente kann eine Mehrfachumformung des Materials zweckmäßig sein, um eine besonders feinkörnige Materialstruktur zu generieren, welche für hohe Belastungen geeignet ist.

Eine Herstellung des Implantats bzw. von Teilen davon, insbesondere des Verbindungselementes und/oder des zumindest einen Ankerelementes, kann durch übliche dem Fachmann bekannte Fertigungsverfahren, insbesondere durch Gießverfahren und/oder Strangpressen und/oder spanende Fertigungsverfahren erfolgen. Abhängig von der herzustellenden Form und dem eingesetzten Material kann ein Formgießen, Blockgießen und/oder Stranggießen sowie anschließende spanende Bearbeitung, zum Beispiel durch Fräsen und/oder Drehen, praktikabel sein. Bevorzugt kann der Einsatz eines Druckguss- oder Spritzgussverfahrens zweckmäßig sein, um formgenaue Gussstücke, insbesondere mit kleinen Ausdehnungen und/oder Wandstärken herzustellen. Es hat sich bewährt, Teile des Implantates durch ein Gießverfahren mit anschließendem Strangpressen herzustellen, wodurch eine Herstellung zeitoptimiert und kostengünstig ermöglich ist. Vorteilhaft kann durch das Verwenden von Dauerformen, zum Beispiel im Druckguss oder im Thixomolding, ein hoher Stückzahldurchsatz erreicht werden. Ein Einstellen von bevorzugten Materialeigenschaften, insbesondere bei metallischen Legierungen, kann durch Wärmebehandlung und/oder Umformen erreicht werden. Um den Herstellungsprozess zu optimieren, kann es außerdem vorteilhaft sein, Prozessabläufe zu simulieren. Insbesondere der Einsatz von Finite-Elemente-Methoden ermöglicht ein effizientes Abstimmen von Prozessparametern. Vorteilhaft kann es auch sein, Teile des Implantates durch Sintern herzustellen. Dies ermöglicht ein abgestimmtes Einstellen von Materialeigenschaften abhängig von einer angewendeten Temperaturbehandlung und bietet den Vorteil, eine Porosität im Material gezielt einstellen zu können.

Besonders vorteilhaft kann es sein, wenn das Verbindungselement und/oder das zumindest eine Ankerelement mit einem 3-D-Druck-Verfahren hergestellt werden. Dadurch ist es möglich, auch komplexere Formen herzustellen, welche auf einen spezifischen Behandlungsfall optimiert und angepasst werden können. Eine Designanpassung ist kostengünstig mit wenig Aufwand durchführbar. Auch erfordert ein mit einem 3-D-Druck-Verfahren hergestelltes Element relativ wenig Nachbearbeitung. Vorteilhaft kann weiter gesehen werden, dass durch einen schichtweisen Herstellungsprozess relativ einfach Bereiche mit unterschiedlichen Eigenschaften, insbesondere unterschiedlichen Dichten, in ein Element eingearbeitet werden können. Des Weiteren können insbesondere komplexe Hohlstrukturen mit einem optimierten Stabilitäts-Gewichts-Verhältnis bzw. Stabilitäts-Volumen-Verhältnis mit wenig Aufwand hergestellt werden.

Der Begriff 3-D-Druck-Verfahren umfasst dabei die dem Fachmann bekannten zugehörigen Verfahren, insbesondere die Verfahren Multi-Jet-Modeling, Fused-Deposition-Modeling, selektives Lasersintern sowie Elektronenstrahlsintern.

Um ein verlässliches Implantat kostengünstig herzustellen, hat es sich bewährt, dass das Verbindungselement und/oder das zumindest eine Ankerelement mit einem Gießverfahren und/oder Strangpressen und/oder spanenden Fertigungsverfahren und/oder 3-D-Druck-Verfahren hergestellt werden.

Weitere Merkmale, Vorteile und Wirkungen ergeben sich aus den nachfolgend dargestellten Ausführungsbeispielen. In den Zeichnungen, auf welche dabei Bezug genommen wird, zeigen:
Fig. 1 eine schematische Darstellung eines Einsatzes eines erfindungsgemäßen zweiteiligen Verbindungselementes;
Fig. 2 ein erfindungsgemäßes zweiteiliges Verbindungselement mit einem Bajonettverschluss;
Fig. 3 einen Querschnitt eines Teiles eines erfindungsgemäßen Verbindungselementes, welches eine profilierte Oberfläche aufweist;
Fig. 4 ein erfindungsgemäßes zweiteiliges Verbindungselement mit zwei Durchgangsöffnungen;
Fig. 5 einen Querschnitt eines Teiles eines erfindungsgemäßen Verbindungselementes, welches eine profilierte Oberfläche aufweist;
Fig. 6 zeigt ein erfindungsgemäßes Verbindungselement, welches einteilig ausgeführt ist;
Fig. 7 einen Querschnitt des in Fig. 6 dargestellten Verbindungselementes;
Fig. 8 ein erfindungsgemäßes zweiteiliges Verbindungselement mit einem Schnappverschluss;
Fig. 9a, Fig. 9b sowie Fig. 10a bis Fig. 10c zeigen Varianten von in einen Röhrenknochen einschraubbaren Ankerelementen;
Fig. 11a und Fig. 11b zeigen Varianten von Ankerelementen, welche mit einer knopfähnlichen Form ausgebildet sind.

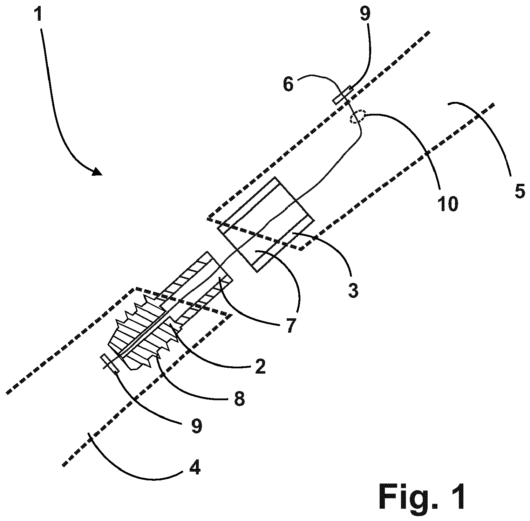

Fig. 1 zeigt eine schematische Darstellung des Einsatzes einer Variante eines erfindungsgemäßen zweiteiligen Verbindungselementes, umfassend ein erstes Teil 2 des Verbindungselementes und ein zweites Teil 3 des Verbindungselementes. Durch die zweiteilige Ausbildung des Verbindungselementes ist ein Einbringen des Verbindungselementes in einen gebrochenen Röhrenknochen durch einen Frakturspalt hindurch erleichtert und ermöglicht einen möglichst minimal-invasiven Eingriff mit einer reduzierten Schädigung des umliegenden Gewebes, insbesondere des Periosts des Röhrenknochens. Hierzu wird das erste Teil 2 des Verbindungselementes durch den Frakturspalt hindurch in ein erstes Teil 4 des gebrochenen Röhrenknochens eingefügt und das zweite Teil 3 des Verbindungselementes durch den Frakturspalt hindurch in ein zweites Teil 5 des gebrochenen Röhrenknochens eingefügt. Werden die beiden Teile 2, 3 des Verbindungselementes miteinander verbunden, ist eine intramedulläre Bruchstabilisierung geschaffen, wodurch wirkenden Belastungen, beispielsweise Translations-, Biege- und/oder Kippkräfte, aufgenommen und weitergeleitet werden können. Um außerdem eine relative Verschiebung der Röhrenknochenteile 4, 5 in axialer Richtung zu unterbinden und zusätzlich eine Kompressionskraft auf die Bruchstelle aufzubringen und damit die Knochenheilung zu stimulieren, ist vorgesehen, dass die beiden gebrochenen Röhrenknochenteile 4, 5 mit zumindest einem Faden 6 in axialer Richtung aneinander gespannt werden. Hierzu weist das Verbindungselement bzw. die beiden Teile 2, 3 des Verbindungselementes eine Durchgangsöffnung 7 auf, durch welche der Faden 6 durchgeführt ist. Der Faden 6 verläuft dabei in Knochenachsenrichtung und mittig durch einen Querschnitt des Verbindungselementes. Der erste Teil 2 des Verbindungselementes ist dabei in durch ein selbstschneidendes Gewinde 8 im ersten Teil 4 des gebrochenen Röhrenknochens fixiert, wodurch eine stabile, positionsbeständige Fixierung des Verbindungselementes im Bereich der Bruchstelle gewährleistet und die Gefahr einer möglichen Verschiebung des Verbindungselementes im Röhrenknochen vermieden ist. Durch die Fixierung des ersten Teiles 2 des Verbindungselementes im ersten Teil 4 des Röhrenknochens kann ein axiales Spannen der Röhrenknochenteile 4, 5 aneinander schon dadurch erreicht werden, dass das erste Teil 2 des Verbindungselementes mit dem Faden 6 an das zweite Röhrenknochenteil 5 gespannt wird. Hierzu ist der Faden 6 mit dessen einem Ende im ersten Teil 4 des Röhrenknochens an einem Ankerelement 9 fixiert, ist weiter durch die Durchgangsöffnungen 7 der beiden Teile 2, 3 des Verbindungselementes geführt und wird im zweiten Teil 5 des Röhrenknochens durch ein in den zweiten Teil 5 des Röhrenknochens eingebrachtes Bohrloch 10 nach außen geführt. Das axiale Spannen der Röhrenknochenteile 4, 5 kann nun einfach durch Zug am nach außen geführten Ende des Fadens 6 erreicht und durch Fixierung des nach außen geführten Endes des Fadens 6, etwa an einem Ankerelement 9, aufrechterhalten werden. Dies ermöglicht ein effizientes Stabilisieren und Aneinanderspannen der gebrochenen Röhrenknochenteile 4, 5 mit reduziertem operativem Aufwand, da der Faden 6 nur an einem der Röhrenknochenteile 4, 5 befestigt werden muss.

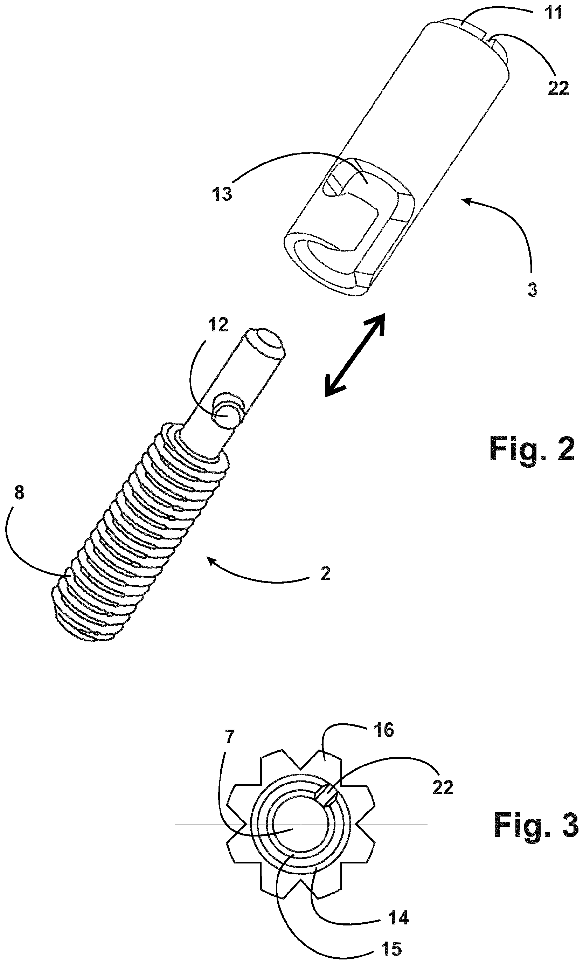

Fig. 2 zeigt eine weitere Variante eines erfindungsgemäßen zweiteiligen Verbindungselementes, umfassend ein erstes Teil 2 des Verbindungselementes, und ein zweites Teil 3 des Verbindungselementes. Das erste Teil 2 des Verbindungselementes weist ein Gewinde 8 auf, um im ersten Teil 4 des gebrochenen Röhrenknochens fixiert zu werden. Das zweite Teil 3 des Verbindungselementes ist derart ausgeführt, dass dieses einfach in das zweite Teil 5 des Röhrenknochens eingeführt werden kann und ist hierzu insbesondere mit einem abgeschrägten Ende 11 ausgebildet. Die beiden Teile 2, 3 des Verbindungselementes sind mit einem Bajonettverschluss lösbar verbindbar ausgeführt, um ein einfaches und schnelles Verbinden zu ermöglichen. Das erste Teil 2 des Verbindungselementes weist hierzu einen Verriegelungszapfen 12 auf, welcher normal zur Verbindungsrichtung ausgeformt ist und in eine korrespondierende Führung 13 am zweiten Teil 3 des Verbindungselementes einfügbar ist. Die Führung 13 am zweiten Teil 3 des Verbindungselementes ist als L-ähnliche Ausnehmung ausgebildet, und zwar mit einer in Richtung der Verbindungsrichtung ausgeformten Längsausnehmung, welche in eine im Wesentlichen normal zur Verbindungsrichtung ausgeformte Querausnehmung übergeht. Damit können die beiden Teile 2, 3 des Verbindungselementes mit einer einfach auszuführenden Steck-Dreh-Bewegung verbunden werden. An die Querausnehmung anschließend ist eine kurze weitere Längsausnehmung ausgebildet, welche im Wesentlichen entgegen der Richtung der Verbindungsrichtung geformt ist. Diese stellt die vorgesehene Verschlussposition des Verriegelungszapfens 12 dar, wodurch eine ausgeprägte form- und kraftschlüssige Fixierung des Verriegelungszapfens 12 erreicht werden kann, welche insbesondere bei Zugbelastung eine ungewollte Lockerung oder ein ungewolltes Öffnen des Bajonettverschlusses verhindert.

Um eine relative Verschiebung der Röhrenknochenteile 4, 5 in axialer Richtung zu unterbinden und zusätzlich eine Kompressionskraft auf die Bruchstelle zur Stimulierung der Knochenheilung aufzubringen, ist vorgesehen, dass die beiden gebrochene Röhrenknochenteile 4, 5 mit zumindest einem Faden 6 in axialer Richtung aneinander gespannt werden. Hierzu ist vorgesehen, dass das Verbindungselement bzw. eines der Teile 2, 3 des Verbindungselementes zumindest eine Durchgangsöffnung 7 aufweist, durch welche der zumindest eine Faden 6 durchführbar ist, dies ist in Fig. 2 nicht dargestellt. Der zumindest eine Faden 6 kann dabei einfach oder mehrfach verlegt sein und verläuft vorzugsweise in Knochenachsenrichtung und mittig durch einen Querschnitt des Verbindungselementes. Das zweite Teil 3 des Verbindungselementes weist zudem eine Führungsrille 22 auf, durch welche der zumindest eine Faden führbar ist, um das Verbindungselement bzw. das zweite Teil 3 des Verbindungselementes rotationsfest im Röhrenknochen zu positionieren.

Fig. 3 zeigt einen Querschnitt eines erfindungsgemäßen Teiles 2, 3 eines Verbindungselementes. Das Verbindungselementteil weist eine Durchgangsöffnung 7 auf, welche zentral durch das Verbindungselementteil geführt ist. Weiter ist zumindest ein Ende des Verbindungselementteiles sowohl außenseitig als auch innenseitig abgeschrägt und weist daher außenseitig ein konisch zulaufendes Ende 14 und innenseitig ein konisch öffnendes Ende 15 auf. Dies erleichtert einerseits das Einfügen der Teile 2, 3 des Verbindungselementes in einen Röhrenknochenteil 4, 5 und sorgt außerdem für eine gleitende Durchführung des Fadens 6 durch die Durchgangsöffnung 7. Ersichtlich ist außerdem eine in einem Endbereich des Verbindungselementteiles angeordnete Führungsrille 22, durch welche der Faden geführt werden kann, um das Verbindungselement bzw. das Verbindungselementteil rotationsfest im Röhrenknochen zu positionieren. Das Verbindungselementteil weist außerdem eine profilierte Oberfläche in Form von Längsrippen 16 auf, wodurch eine drehfeste Positionierung im Röhrenknochen erreicht wird. Die Längsrippen 16 weisen im Querschnitt eine trapezähnliche Form auf, wodurch eine ausgeprägte Verzahnung in einem Markraum eines Röhrenknochenteiles 4, 5 erreicht wird.

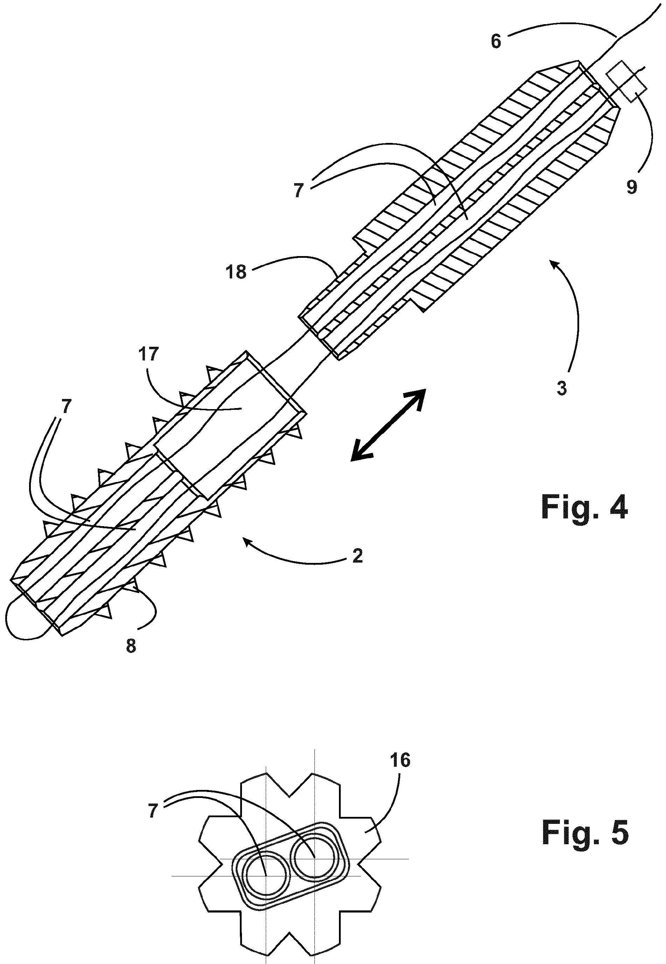

Fig. 4 zeigt eine weitere Variante eines erfindungsgemäßen zweiteiligen Verbindungselementes, umfassend ein erstes Teil 2 des Verbindungselementes, und ein zweites Teil 3 des Verbindungselementes, wobei das Verbindungselement zwei Durchgangsöffnungen 7 aufweist, welche sich in Längsrichtung des Verbindungselementes im Wesentlichen durch beide Teile 2, 3 des Verbindungselementes erstrecken. Die beiden Teile 2, 3 des Verbindungselementes sind durch ein Stecken verbindbar. Hierzu weist das erste Teil 2 des Verbindungselementes eine Steckeraufnahme 17 auf, in welche ein Steckerbereich 18 des zweiten Teiles 3 des Verbindungselementes einführbar ist. Hierdurch ist ermöglicht, dass die beiden Teile 2, 3 des Verbindungselementes mit einer Durchführung eines Fadens 6 durch die beiden Durchgangsöffnungen 7 aneinander anspannbar sind. Das erste Teil 2 des Verbindungselementes ist außerdem mit einem Gewinde 8 ausgestattet, um ein Einschrauben und eine positionsbeständige Fixierung in einem Teil 3, 4 des Röhrenknochens sicherzustellen. Es ist möglich die beiden Teile 2, 3 des Verbindungselementes mit einem durch die beiden Durchgangsöffnungen 7 durchgeführten Faden 6 zu verbinden und zum Spannen der beiden Teile 4, 5 des Röhrenknochens einen weiteren Faden 6 zu verwenden. Zweckmäßig ist es jedoch, wenn zum Spannen der beiden Teile 4, 5 des Röhrenknochens und zum Aneinanderspannen der beiden Teile 2, 3 des Verbindungselementes derselbe Faden 6 verwendet wird. In Fig. 4 ist hierzu der Faden 6 derart durch die zwei Durchgangsöffnungen 7 der beiden Teile 2, 3 des Verbindungselementes durchgeführt, dass beide Fadenenden auf jener Seite zu liegen kommen, von welcher aus die beiden Knochenteile 4, 5 aneinander gespannt werden können. Indem ein erstes Fadenende mit einem Ankerelement 9 fixiert ist, können durch Zugbelastung des anderen, zweiten Fadenendes sowohl die beiden Verbindungselementteile 2, 3 aneinander als auch das in einem Teil 4, 5 des Röhrenknochens fixierte erste Teil 2 des Verbindungselementes an den anderen Teil 4, 5 des Röhrenknochens gespannt werden. Durch das Verbinden der beiden Teile 2, 3 des Verbindungselementes mit einer Steckverbindung, welche ein Aneinanderspannen der beiden Teile 2, 3 des Verbindungselementes mit einem Faden vorsieht, ist eine einfache Ausgestaltung des Verbindungselementes verwirklicht, da auf einen zusätzlichen Verschluss zum beständigen Verbinden der beiden Teile 2, 3 des Verbindungselementes verzichtet werden kann.

Fig. 5 zeigt einen Querschnitt eines erfindungsgemäßen Teiles 2, 3 eines Verbindungselementes, welches zwei Durchgangsöffnungen 7 und eine profilierte Oberfläche mit trapezförmigen Längsrippen 16 aufweist.

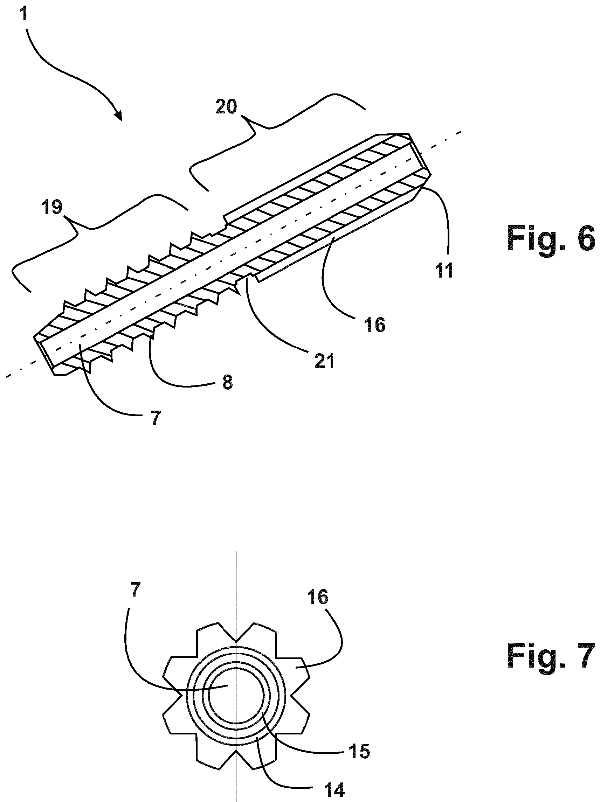

Fig. 6 zeigt ein erfindungsgemäßes Verbindungselement, welches einteilig ausgeführt ist. Dieses weist in einem ersten Bereich 19 ein Gewinde 8 auf, um in einem Teil 4, 5 eines Röhrenknochens fixiert zu werden. Ein zweiter Bereich 20 ist mit einer profilierten Oberfläche, im Speziellen mit Längsrippen 16 ausgestattet, um nach einem Einfügen in einen anderen Teil 4, 5 des Röhrenknochens eine drehfeste Verankerung zu erreichen. Ein Abschnitt des Verbindungselementes ist mit einer Mehrkantoberfläche 21 ausgebildet, um ein Einschrauben des Verbindungselementes mit einem Gabelschlüssel zu ermöglichen. Das Verbindungselement ist derart ausgeführt, dass dieses einfach in die Röhrenknochenteile 4, 5 eingefügt werden kann und ist hierzu insbesondere mit abgeschrägten Enden 11 ausgebildet.

Fig. 7 zeigt eine Ansicht in Richtung der Rotationsachse des Verbindungselementes nach Fig. 6. Die Längsrippen 16 sind zur drehfesten Verankerung mit einem trapezförmigen Querschnitt ausgebildet, wodurch eine ausgeprägte Verzahnung in einem Markraum eines Röhrenknochenteiles 4, 5 erreicht wird. Zentral durch das Verbindungselement verläuft eine Durchgangsöffnung 7, um eine hindernisfreie Durchführung des Fadens 6 zu gewährleisten, welcher die beiden Röhrenknochenteile 4, 5 zugfest spannt. Weiter ist das Ende des Verbindungselementes sowohl außenseitig als auch innenseitig abgeschrägt und weist daher außenseitig ein konisch zulaufendes Ende 14 und innenseitig ein konisch öffnendes Ende 15 auf. Dies erleichtert einerseits das Einfügen des Verbindungselementes in einen Röhrenknochenteil 4, 5 und sorgt außerdem für eine gleitende Durchführung des Fadens 6 durch die Durchgangsöffnung 7.

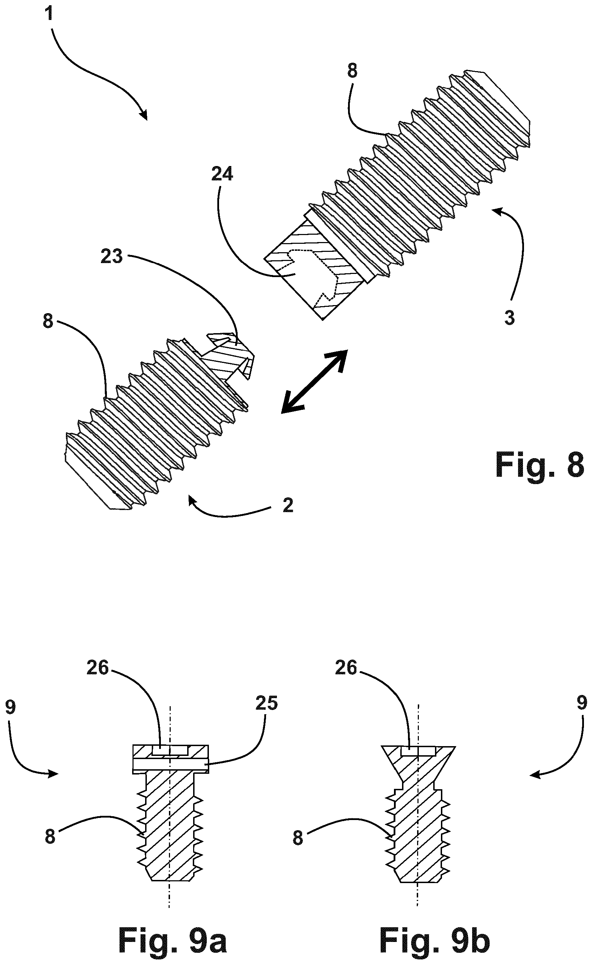

Fig. 8 zeigt eine weitere Variante eines erfindungsgemäßen zweiteiligen Verbindungselementes, umfassend ein erstes Teil 2 des Verbindungselementes, und ein zweites Teil 3 des Verbindungselementes. Sowohl das erste Teil 2 als auch das zweite Teil 3 weisen selbstschneidende Gewinde 8 auf, sodass das erste Teil 2 des Verbindungselementes in einem Teil 4, 5 des gebrochenen Röhrenknochens unmittelbar fixierbar ist und das zweite Teil 3 des Verbindungselementes im anderen Teil 4, 5 des gebrochenen Röhrenknochens unmittelbar fixierbar ist. Dadurch ist auf einfache Weise eine kraftschlüssige Fixierung der Teile des Verbindungselementes 2, 3 in den Röhrenknochenteilen 4, 5 erreichbar. Die beiden Teile 2, 3 des Verbindungselementes sind mit einem Schnappverschluss verbindbar ausgeführt, um ein einfaches und schnelles direktes Verbinden der Teile 2, 3 zu ermöglichen. Das erste Teil 2 des Verbindungselementes weist hierzu ein Steckelement 23 auf, welches in eine Steckelementaufnahme 24 am zweiten Teil 3 des Verbindungselementes einfügbar ist. Das Steckelement 23 weist einen elastisch verformbaren Kopfbereich auf, welcher sich bei einem Einfügen in die Steckelementaufnahme 24 elastisch verformt und formschlüssig verhakt. Dadurch wird auf einfache Weise eine robuste Verbindung der beiden Teile 2, 3 des Verbindungselementes und damit auch der Röhrenknochenteile 4, 5 hergestellt.

Fig. 9a und Fig. 9b zeigen zwei Varianten eines erfindungsgemäßen in einen Röhrenknochen einschraubbaren Ankerelementes 9. Einschraubbare Ankerelemente 9 bieten den Vorteil, dass ein Einschraubvorgang des Ankerelementes 9 gleichzeitig zum Spannen eines am Ankerelement 9 befestigten Fadens 6 verwendet werden kann. Der am Ankerelement 9 befestigte Faden 6 wird durch den Einschraubvorgang um das Ankerelement 9 gewickelt und dadurch gespannt.

Fig. 9a zeigt ein Ankerelement 9, welches als Zylinderkopfschraube ausgebildet ist. Das Gewinde 8 der Zylinderkopfschraube ist selbstschneidend ausgeführt, um eine einfache Fixierung im Röhrenknochen zu ermöglichen. Im oberen Bereich weist die Zylinderkopfschraube einen Durchgangskanal 25 auf, durch welchen ein zu befestigender bzw. zu spannender Faden 6 durchgeführt werden kann. Ein Befestigen bzw. Spannen des Fadens 6 wird dadurch vereinfacht und ein Abrutschen bzw. Lösen des Fadens 6 unterbunden. In oberen Bereich der Zylinderkopfschraube ist zudem eine Werkzeugaufnahme 26 dargestellt, welche als Sechskantform ausgeführt ist, um ein Eindrehen der Zylinderkopfschraube mit einem Werkzeug, etwa einem Sechskantschraubenschlüssel, zu ermöglichen.

Fig. 9b zeigt ein Ankerelement 9, welches als Senkkopfschraube ausgebildet ist. Eine konische Form eines Kopfbereiches der Senkkopfschraube ermöglicht es, dass bei einem Eindrehen in den Röhrenknochen auch der Kopfbereich der Senkkopfschraube zumindest teilweise im Röhrenknochen versenkt wird. Um einen Faden 6 mit der Senkkopfschraube zu spannen, ist vorgesehen, dass der Faden 6 um einen oberen Bereich der Senkkopfschraube gewickelt wird und im Zuge des Eindrehens in den Röhrenknochen vorzugsweise gemeinsam mit dem Kopfbereich der Senkkopfschraube im Röhrenknochen versenkt wird. Dadurch ist eine belastbare Fixierung des Fadens 6 erreicht.

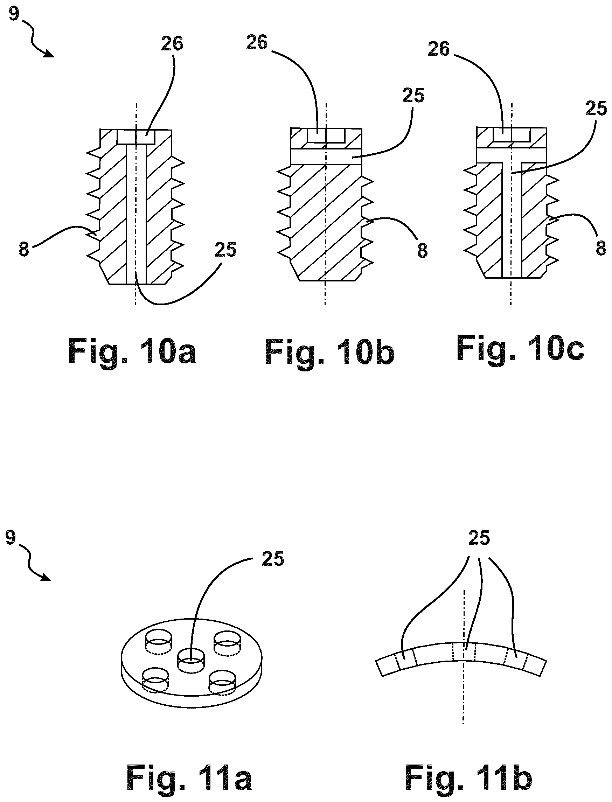

Fig. 10a bis Fig 10c zeigen weitere Varianten von in einen Röhrenknochen einschraubbaren Ankerelementen 9, wobei diese kopflos ausgebildet sind, sodass diese bei einem Eindrehen in den Röhrenknochen gänzlich im Röhrenknochen versenkt werden können. Die Ankerelemente 9 weisen Durchgangskanäle 25 auf, durch welche ein Faden 6 zur Befestigung bzw. zum Spannen durchgeführt werden kann.

Das in Fig. 10a dargestellte Ankerelement 9 weist einen entlang einer Einschraubrichtung verlaufenden Durchgangskanal 25 auf. Dadurch kann das Ankerelement 9 in ein Bohrloch 10, welches in einen Röhrenknochen eingebracht wurde, um einen Faden 6 vom Markraum des Röhrenknochens nach außen zu führen, eingefügt bzw. eingeschraubt werden. Der Faden 6 kann dabei durch den Durchgangskanal 25 des Ankerelementes 9 nach außen geführt und am Ankerelement 9 befestigt werden.

Fig. 10b zeigt ein Ankerelement 9 mit einem im Wesentlichen vertikal zu einer Einschraubrichtung verlaufenden Durchgangskanal 25. Ein durch den Durchgangskanal 25 durchgeführter Faden 6 kann damit auf einfache Weise gemeinsam mit dem Ankerelement 9 in einem Röhrenknochen versenkt und dadurch fixiert bzw. gespannt werden.

Fig. 10c zeigt ein Ankerelement 9 mit einem in Einschraubrichtung verlaufenden Durchgangskanal 25, welcher in einem oberen Bereich des Ankerelementes 9 in einen im Wesentlichen vertikal zur Einschraubrichtung verlaufenden weiteren Durchgangskanal 25 mündet, sodass die Durchgangskanäle 25 einen T-förmigen Verlauf bilden. Damit werden die Vorteile der in Fig. 10a und Fig. 10b dargestellten Ankerelemente 9 kombiniert. Es versteht sich, dass die vorgenannten Vorteile alleine dadurch erreicht sind, dass ein Ankerelement 9 ein oder mehrere Durchgangskanäle 25 aufweist, sodass zumindest ein Faden 6 derart durch das Ankerelement 9 durchführbar ist, dass der zumindest eine Faden 6 in einem unteren Bereich des Ankerelementes 9 in das Ankerelement 9 eintritt und in einem oberen Bereich seitlich am Ankerelement 9 wieder austritt. Zweckmäßig sind Durchgangskanäle 25 dabei derart angeordnet, dass starke Krümmungen vermieden sind, um eine Fadenführung entlang ausgeprägter Kanten zu vermeiden. Dementsprechend können Durchgangskanäle 25 etwa auch einen Y-förmigen Verlauf bilden, um dadurch eine reibungsarme Fadendurchführung zu ermöglichen.

Fig. 11a und Fig. 11b stellen Varianten eines Ankerelementes 9 dar, welche mit einer knopfähnlichen Form ausgebildet sind. Zur Befestigung bzw. zum Spannen zumindest eines Fadens 6 weisen auch diese Ankerelemente 9 Durchgangskanäle 25 auf. Eine knopfähnliche Form weist ein kleines Volumen auf und stellt bei flächigem Anlegen an eine Knochenoberfläche aufgrund dessen geringen Höhe nur eine kleine Beeinträchtigung der Umgebung dar. Ein flächiges Anlegen an eine gekrümmte Oberfläche eines Röhrenknochens ist dadurch optimiert, dass das knopfähnliche Ankerelement 9 gebogen bzw. gekrümmt ausgebildet ist. Dies ist exemplarisch in Fig. 11b dargestellt, welches eine Seitenansicht eines gekrümmt geformten Ankerelementes 9 zeigt. Damit kann ein Faden 6 auf einfache Weise, mit wenig Beeinträchtigung der Umgebung befestigt und gespannt werden.

## Patentansprüche

1. Implantat (1) zum zugfesten Verbinden zumindest zweier Teile (4, 5) eines gebrochenen Röhrenknochens, umfassend ein Verbindungselement (2, 3), zumindest einen Faden (6) und zumindest ein Ankerelement (9), wobei das zumindest eine Ankerelement (9) mit dem zumindest einen Faden (6) an einem Knochenteil (4, 5) befestigbar ist, wobei das Verbindungselement derart ausgeführt ist, dass dieses in Längsrichtung des gebrochenen Röhrenknochens in einen Markraum des gebrochenen Röhrenknochens einführbar ist, wobei das Verbindungselement zumindest eine Durchgangsöffnung (7) aufweist, durch welche der zumindest eine Faden (6) zum zugfesten Spannen der Röhrenknochenteile (4, 5) durchführbar ist, wobei das Verbindungselement zweiteilig ausgebildet ist, **dadurch gekennzeichnet, dass** das Verbindungselement und/oder das zumindest eine Ankerelement und/oder der zumindest eine Faden zumindest teilweise aus einer Magnesiumbasislegierung gebildet sind, wobei die Legierung in Gew.-% enthält:
mehr als 0,0 bis 5,0 % Zn,
mehr als 0,0 bis 1,0 % Ca,
optional mehr als 0,0 bis 2,0 % Mn,
Rest Mg und herstellungsbedingte Verunreinigungen, um durch eine abgestimmte Wärmebehandlung der Legierung über ein Verhältnis von Mg₂Ca-Phasen und Ca₂Mg₆Zn₃-Phasen in der Legierung deren Degradationsrate einstellen zu können.

2. Implantat (1) nach Anspruch 1, **dadurch gekennzeichnet, dass** ein erstes Teil (2) des Verbindungselementes in einem Teil (4, 5) des gebrochenen Röhrenknochens fixierbar ist und ein zweites Teil (3) des Verbindungselementes in das andere Teil (4, 5) des gebrochenen Röhrenknochens einführbar ist.

3. Implantat (1) nach Anspruch 2, **dadurch gekennzeichnet, dass** das erste Teil (2) des Verbindungselementes ein Gewinde (8) aufweist, insbesondere ein selbstschneidendes Gewinde (8), mit welchem dieses im gebrochenen Röhrenknochen fixierbar ist.

4. Implantat (1) nach einem der Ansprüche 1 bis 3, **dadurch gekennzeichnet, dass** die beiden Teile (2, 3) des Verbindungselementes durch eine Schraubverbindung oder einen Verschluss, insbesondere einen Bajonettverschluss oder einen Schnappverschluss, miteinander verbindbar ausgeführt sind.

5. Implantat (1) nach einem der Ansprüche 1 bis 4, **dadurch gekennzeichnet, dass** zumindest ein Abschnitt des Verbindungselementes mit einer Mehrkantoberfläche (21) ausgebildet ist, um ein Eindrehen des Verbindungselementes bzw. eines Teiles (2, 3) des Verbindungselementes mit einem Werkzeug zu ermöglichen.

6. Implantat (1) nach einem der Ansprüche 1 bis 5, **dadurch gekennzeichnet, dass** das Verbindungselement länglich, insbesondere im Wesentlichen zylinderförmig oder spindelförmig, geformt ist.

7. Implantat (1) nach einem der Ansprüche 1 bis 6, **dadurch gekennzeichnet, dass** das Verbindungselement zumindest bereichsweise eine profilierte Oberfläche aufweist.

8. Implantat (1) nach einem der Ansprüche 1 bis 7, **dadurch gekennzeichnet, dass** das Verbindungselement zumindest teilweise mit Längsrippen (16) versehen ist.

9. Implantat (1) nach einem der Ansprüche 1 bis 8, **dadurch gekennzeichnet, dass** sich die zumindest eine Durchgangsöffnung (7) des Verbindungselementes in Längsrichtung des Verbindungselementes durch das gesamte Verbindungselement erstreckt, um eine Durchführung des Fadens (6) durch das gesamte Verbindungselement zu ermöglichen.

10. Implantat (1) nach einem der Ansprüche 1 bis 9, **dadurch gekennzeichnet, dass** das Verbindungselement als Hohlstruktur oder Hohlkammerstruktur, insbesondere als Hohlzylinder, ausgebildet ist.

11. Implantat (1) nach einem der Ansprüche 1 bis 10, **dadurch gekennzeichnet, dass** die Enden der beiden Teile (2, 3) des Verbindungselementes verjüngt ausgebildet sind.

12. Implantat (1) nach einem der Ansprüche 1 bis 11, **dadurch gekennzeichnet, dass** die beiden Verbindungselementteile durch ein Stecken formschlüssig verbindbar sind und das Verbindungselement zumindest zwei Durchgangsöffnungen (7) aufweist, welche sich in Längsrichtung des Verbindungselementes, insbesondere im Wesentlichen parallel, durch das gesamte Implantat (1) erstrecken, wodurch mit einer Durchführung eines Fadens (6) durch die beiden Durchgangsöffnungen (7) die beiden Teile (2, 3) des Verbindungselementes aneinander anspannbar sind.

13. Implantat (1) zum zugfesten Verbinden zweier Teile (4, 5) eines gebrochenen Röhrenknochens, umfassend ein Verbindungselement, zumindest einen Faden (6) und zumindest ein Ankerelement (9), wobei das zumindest eine Ankerelement (9) mit dem zumindest einen Faden (6) an einem Knochenteil (4, 5) befestigbar ist, wobei das Verbindungselement derart ausgeführt ist, dass dieses in Längsrichtung des gebrochenen Röhrenknochens in den Markraum des gebrochenen Röhrenknochens einführbar ist, wobei das Verbindungselement zumindest eine Durchgangsöffnung (7) aufweist, durch welche der zumindest eine Faden (6) zum zugfesten Spannen der Röhrenknochenteile (4, 5) durchführbar ist, **dadurch gekennzeichnet, dass** das Verbindungselement einteilig ausgebildet ist und aus einer resorbierbaren Magnesiumbasislegierung gebildet ist, wobei die Legierung in Gew.-% enthält:
mehr als 0,0 bis 5,0 % Zn,
mehr als 0,0 bis 1,0 % Ca,
optional mehr als 0,0 bis 2,0 % Mn,
Rest Mg und herstellungsbedingte Verunreinigungen,
um durch eine abgestimmte Wärmebehandlung der Legierung über ein Verhältnis von Mg₂Ca-Phasen und Ca₂Mg₆Zn₃-Phasen in der Legierung deren Degradationsrate einstellen zu können.

14. Implantat zum zugfesten Verbinden zumindest zweier Teile (4, 5) eines gebrochenen Röhrenknochens, umfassend ein Verbindungselement (2, 3), wobei das Verbindungselement derart ausgeführt ist, dass dieses in Längsrichtung des gebrochenen Röhrenknochens in einen Markraum des gebrochenen Röhrenknochens einführbar ist, wobei das Verbindungselement zweitteilig ausgeführt ist, wobei ein erstes Teil (2) des Verbindungselementes in einem Teil (4, 5) des gebrochenen Röhrenknochens unmittelbar fixierbar ist und ein zweites Teil (3) des Verbindungselementes im anderen Teil (4, 5) des gebrochenen Röhrenknochens unmittelbar fixierbar ist, wobei das erste Teil (2) mit dem zweiten Teil (3) zur Heilung des gebrochenen Röhrenknochens verbindbar ist, **dadurch gekennzeichnet, dass** das Verbindungselement zumindest teilweise aus einer Magnesiumbasislegierung gebildet ist, wobei die Legierung in Gew.-% enthält:
mehr als 0,0 bis 5,0 % Zn,
mehr als 0,0 bis 1,0 % Ca,
optional mehr als 0,0 bis 2,0 % Mn,
Rest Mg und herstellungsbedingte Verunreinigungen,
um durch eine abgestimmte Wärmebehandlung der Legierung über ein Verhältnis von Mg₂Ca-Phasen und Ca₂Mg₆Zn₃-Phasen in der Legierung deren Degradationsrate einstellen zu können.

15. Verfahren zur Herstellung eines Implantates (1) nach einem der Ansprüche 1 bis 14, **dadurch gekennzeichnet, dass** ein Equal-channel-angular-pressing-Verfahren angewendet wird.

16. Verfahren zur Herstellung eines Implantates (1) nach einem der Ansprüche 1 bis 14, **dadurch gekennzeichnet, dass** das Verbindungselement und/oder das zumindest eine Ankerelement (9) mit einem Gießverfahren und/oder Strangpressen und/oder spanenden Fertigungsverfahren und/oder 3-D-Druck-Verfahren hergestellt werden.

## Claims

1. An implant (1) for extension-resistant connection of at least two parts (4, 5) of a broken long bone, comprising a connecting element (2, 3), at least one filament (6) and at least one anchor element (9), wherein the at least one anchor element (9) can be fastened to a bone part (4, 5) with the at least one filament (6), wherein the connecting element is configured in a manner such that it can be inserted into a medullary space of the broken long bone in the longitudinal direction of the broken long bone, wherein the connecting element has at least one through opening (7) through which the at least one filament (6) can be passed for extension-resistant bracing of the parts of the long bone (4, 5), wherein the connecting element has a two-part configuration, **characterized in that** the connecting element and/or the at least one anchor element and/or the at least one filament are at least partially formed from a magnesium-based alloy, wherein the alloy contains, as a % by weight:
more than 0.0 to 5.0% Zn,
more than 0.0 to 1.0% Ca,
optionally, more than 0.0 to 2.0% Mn,
the remainder being Mg and impurities due to the manufacturing conditions,
in order, by employing an appropriate heat treatment of the alloy, to be able to adjust the rate of degradation of the alloy via a ratio of Mg₂Ca phases and Ca₂Mg₆Zn₃ phases in the alloy.

2. The implant (1) as claimed in claim 1, **characterized in that** a first portion (2) of the connecting element can be fastened in a part (4, 5) of the broken long bone and a second portion (3) of the connecting element can be inserted into the other part (4, 5) of the broken long bone.

3. The implant (1) as claimed in claim 2, **characterized in that** the first portion (2) of the connecting element has a screw thread (8), in particular a self-tapping screw thread (8), with which it can be fastened in the broken long bone.

4. The implant (1) as claimed in one of claims 1 to 3, **characterized in that** the two portions (2, 3) of the connecting element are configured so as to be capable of being connected together by means of a screw connection or a fastening, in particular a bayonet fastening or a snap fastening.

5. The implant (1) as claimed in one of claims 1 to 4, **characterized in that** at least one section of the connecting element is configured with a polygonal surface (21) in order to enable the connecting element or a portion (2, 3) of the connecting element to be screwed in using a tool.

6. The implant (1) as claimed in one of claims 1 to 5, **characterized in that** the connecting element is elongate in shape, in particular substantially cylindrically shaped or spindle-shaped.

7. The implant (1) as claimed in one of claims 1 to 6, **characterized in that** the connecting element has a profiled surface at least in regions thereof.

8. The implant (1) as claimed in one of claims 1 to 7, **characterized in that** at least a portion of the connecting element is provided with longitudinal ribs (16) .

9. The implant (1) as claimed in one of claims 1 to 8, **characterized in that** the at least one through opening (7) of the connecting element extends in the longitudinal direction of the connecting element through the entire connecting element in order to enable the filament (6) to be passed through the entire connecting element.

10. The implant (1) as claimed in one of claims 1 to 9, **characterized in that** the connecting element is configured as a hollow structure or hollow chamber structure, in particular as a hollow cylinder.

11. The implant (1) as claimed in one of claims 1 to 10, **characterized in that** the ends of both portions (2, 3) of the connecting element are tapered in configuration.

12. The implant (1) as claimed in one of claims 1 to 11, **characterized in that** the two portions of the connecting element can be connected and interlocked by plugging them into each other and the connecting element has at least two through openings (7) which extend in the longitudinal direction of the connecting element, in particular substantially parallel, through the entire implant (1), whereupon by passing a filament (6) through the two through openings (7), the two portions (2, 3) of the connecting element can be tightened against each other.

13. An implant (1) for extension-resistant connection of two parts (4, 5) of a broken long bone, comprising a connecting element, at least one filament (6) and at least one anchor element (9), wherein the at least one anchor element (9) can be fastened to a bone part (4, 5) with the at least one filament (6), wherein the connecting element is configured in a manner such that it can be inserted into the medullary space of the broken long bone in the longitudinal direction of the broken long bone, wherein the connecting element has at least one through opening (7) through which the at least one filament (6) can be passed for extension-resistant bracing of the parts of the long bone (4, 5), **characterized in that** the connecting element has a one-piece configuration and is formed from a resorbable magnesium-based alloy, wherein the alloy contains, as a % by weight:
more than 0.0 to 5.0% Zn,
more than 0.0 to 1.0% Ca,
optionally, more than 0.0 to 2.0% Mn,
the remainder being Mg and impurities due to the manufacturing conditions,
in order, by employing an appropriate heat treatment of the alloy, to be able to adjust the rate of degradation of the alloy via a ratio of Mg₂Ca phases and Ca₂Mg₆Zn₃ phases in the alloy.

14. An implant for extension-resistant connection of at least two parts (4, 5) of a broken long bone, comprising a connecting element (2, 3), wherein the connecting element is configured in a manner such that it can be inserted into a medullary space of the broken long bone in the longitudinal direction of the broken long bone, wherein the connecting element has a two-part configuration, wherein a first portion (2) of the connecting element can be directly fastened in a part (4, 5) of the broken long bone and a second portion (3) of the connecting element can be directly fastened in the other part (4, 5) of the broken long bone, wherein the first portion (2) can be connected to the second portion (3) in order to heal the broken long bone, **characterized in that** the connecting element is at least partially formed from a resorbable magnesium-based alloy, wherein the alloy contains, as a % by weight:
more than 0.0 to 5.0% Zn,
more than 0.0 to 1.0% Ca,
optionally, more than 0.0 to 2.0% Mn,
the remainder being Mg and impurities due to the manufacturing conditions,
in order, by employing an appropriate heat treatment of the alloy, to be able to adjust the rate of degradation of the alloy via a ratio of Mg₂Ca phases and Ca₂Mg₆Zn₃ phases in the alloy.

15. A process for the production of an implant (1) as claimed in one of claims 1 to 14, **characterized in that** an equal channel angular pressing process is employed.

16. A process for the production of an implant (1) as claimed in one of claims 1 to 14, **characterized in that** the connecting element and/or the at least one anchor element (9) is produced by means of a casting process and/or extrusion and/or a machining production process and/or by a 3D printing process.

## Revendications

1. Implant (1) pour la liaison résistante à la traction d'au moins deux parties (4, 5) d'un os long cassé, comprenant un élément de liaison (2, 3), au moins un fil (6) et au moins un élément d'ancrage (9), dans lequel l'au moins un élément d'ancrage (9) peut être fixé à l'au moins un fil (6) sur une partie osseuse (4, 5), dans lequel l'élément de liaison est ainsi conçu que celui-ci peut être introduit dans une cavité médullaire de l'os long cassé dans le sens longitudinal de l'os long cassé, dans lequel l'élément de liaison présente au moins une ouverture traversante (7) à travers laquelle l'au moins un fil (6) peut être passé pour tendre les parties d'os long (4, 5) en résistance à la pression, dans lequel l'élément de liaison est conçu en deux parties, **caractérisé en ce que** l'élément de liaison et/ou l'au moins un élément d'ancrage et/ou l'au moins un fil sont composés au moins partiellement d'un alliage à base de magnésium, dans lequel l'alliage contient en % en poids :
plus de 0,0 à 5,0 % de Zn,
plus de 0,0 à 1,0 % de Ca,
en option, plus de 0,0 à 2,0 % de Mn,
du Mg restant et des impuretés dues à la fabrication,
pour pouvoir régler son taux de dégradation par un traitement thermique adapté de l'alliage par un rapport de phases Mg₂Ca et de phases Ca₂Mg₆Zn₃ dans l'alliage.

2. Implant (1) selon la revendication 1, **caractérisé en ce qu'**une première partie (2) de l'élément de liaison peut être fixée dans une partie (4, 5) de l'os long cassé et une deuxième partie (3) de l'élément de liaison peut être introduite dans l'autre partie (4, 5) de l'os long cassé.

3. Implant (1) selon la revendication 2, **caractérisé en ce que** la première partie (2) de l'élément de liaison présente un filet (8), en particulier un filet auto-taraudeur (8) avec lequel celui-ci peut être fixé dans l'os long cassé.

4. Implant (1) selon l'une des revendications 1 à 3, **caractérisé en ce que** les deux parties (2, 3) de l'élément de liaison sont conçues en pouvant être liées entre elles par un vissage ou un enclenchement, en particulier un enclenchement à baïonnette ou un enclenchement à déclic.

5. Implant (1) selon l'une des revendications 1 à 4, **caractérisé en ce qu'**au moins un tronçon de l'élément de liaison est conçu avec une surface à plusieurs arêtes (21) pour permettre un vissage de l'élément de liaison, respectivement d'une partie (2, 3) de l'élément de liaison avec un outil.

6. Implant (1) selon l'une des revendications 1 à 5, **caractérisé en ce que** l'élément de liaison est conçu longitudinal, en particulier essentiellement cylindrique ou hélicoïdal.

7. Implant (1) selon l'une des revendications 1 à 6, **caractérisé en ce que** l'élément de liaison présente une surface profilée au moins par tronçons.

8. Implant (1) selon l'une des revendications 1 à 7, **caractérisé en ce que** l'élément de liaison est doté au moins partiellement de nervures longitudinales (16).

9. Implant (1) selon l'une des revendications 1 à 8, **caractérisé en ce que** l'au moins une ouverture traversante (7) de l'élément de liaison s'étend dans le sens longitudinal de l'élément de liaison à travers tout l'élément de liaison pour permettre un passage du fil (6) à travers tout l'élément de liaison.

10. Implant (1) selon l'une des revendications 1 à 9, **caractérisé en ce que** l'élément de liaison est conçu en tant que structure creuse ou structure à chambre creuse, en particulier en tant que cylindre creux.

11. Implant (1) selon l'une des revendications 1 à 10, **caractérisé en ce que** les extrémités des deux parties (2, 3) de l'élément de liaison sont conçues rétrécies.

12. Implant (1) selon l'une des revendications 1 à 11, **caractérisé en ce que** les deux parties d'élément de liaison peuvent être reliées entre elles en complémentarité de formes par enfichage et l'élément de liaison présente au moins deux ouvertures traversantes (7) qui s'étendent dans le sens longitudinal de l'élément de liaison, en particulier essentiellement parallèlement, à travers tout l'implant (1), ce par quoi par un passage d'un fil (6) à travers les deux ouvertures traversantes (7), les deux parties (2, 3) de l'élément de liaison peuvent être tendues l'une sur l'autre.

13. Implant (1) pour la liaison résistante à la traction d'au moins deux parties (4, 5) d'un os long cassé, comprenant un élément de liaison, au moins un fil (6) et au moins un élément d'ancrage (9), dans lequel l'au moins un élément d'ancrage (9) peut être fixé à l'au moins un fil (6) sur une partie osseuse (4, 5), dans lequel l'élément de liaison est ainsi conçu que celui-ci peut être introduit dans le sens longitudinal de l'os long cassé dans la cavité médullaire de l'os long cassé, dans lequel l'élément de liaison présente au moins une ouverture traversante (7) à travers laquelle l'au moins un fil (6) peut être passé pour tendre les parties d'os long (4, 5) en résistance à la pression, **caractérisé en ce que** l'élément de liaison est conçu d'une seule pièce et est composé d'un alliage à base de magnésium résorbable, dans lequel l'alliage contient en % en poids :
plus de 0,0 à 5,0 % de Zn,
plus de 0,0 à 1,0 % de Ca,
en option, plus de 0,0 à 2,0 % de Mn,
du Mg restant et des impuretés dues à la fabrication,
pour pouvoir régler son taux de dégradation par un traitement thermique adapté de l'alliage par un rapport de phases Mg₂Ca et de phases Ca₂Mg₆Zn₃ dans l'alliage.

14. Implant pour la liaison résistante à la traction d'au moins deux parties (4, 5) d'un os long cassé, comprenant un élément de liaison (2, 3), dans lequel l'élément de liaison est ainsi conçu que celui-ci peut être introduit dans une cavité médullaire de l'os long cassé dans le sens longitudinal de l'os long cassé, dans lequel l'élément de liaison est conçu en deux parties, dans lequel une première partie (2) de l'élément de liaison peut être fixée directement dans une partie (4, 5) de l'os long cassé et une seconde partie (3) de l'élément de liaison peut être fixée directement dans une autre partie (4, 5) de l'os long cassé, dans lequel la première partie (2) peut être reliée à la deuxième partie (3) pour la guérison de l'os long cassé, **caractérisé en ce que** l'élément de liaison est composé au moins partiellement d'un alliage à base de magnésium, dans lequel l'alliage contient en % en poids :
plus de 0,0 à 5,0 % de Zn,
plus de 0,0 à 1,0 % de Ca,
en option, plus de 0,0 à 2,0 % de Mn,
du Mg restant et des impuretés dues à la fabrication,
pour pouvoir régler son taux de dégradation par un traitement thermique adapté de l'alliage par un rapport de phases Mg₂Ca et de phases Ca₂Mg₆Zn₃ dans l'alliage.

15. Procédé de fabrication d'un implant (1) selon l'une des revendications 1 à 14, **caractérisé en ce qu'**un procédé d'extrusion angulaire à section constante est employé.

16. Procédé de fabrication d'un implant (1) selon l'une des revendications 1 à 14, **caractérisé en ce que** l'élément de liaison et/ou l'au moins un élément d'ancrage (9) est/sont fabriqué(s) par un procédé de coulée et/ou d'extrusion et/ou un procédé de fabrication par enlèvement de copeaux et/ou un procédé d'impression 3D.
